# EUROPEAN PATENT SPECIFICATION

(11) **EP 2 542 236 B1**
(45) Date of publication and mention of the grant of the patent: **26.10.2016**
(21) Application number: 11707324.7
(22) Date of filing: 01.03.2011
(51) Int. Cl.: A61K 31/351, A61P 9/00

(54) **COMBINATIONS COMPRISING 6-BENZYLPHENYL-2- SULFURTERAHYDROPYRAN-3,4,5-TRIOL DERIVATIVES AS INHIBITORS OF SODIUM -GLUCOSE COTRANSPORTERS 1 AND 2 FOR USE IN DIABETIC PATIENTS**
KOMBINATION ENTHALTEND 6-BENZYLPHENYL-2- SULFURTERAHYDROPYRAN-3,4,5-TRIOLDERIVATE ALS HEMMER DER NATRIUM-GLUCOSE-COTRANSPORTER 1 UND 2 ZUR VERWENDUNG BEI DIABETESPATIENTEN
COMBINAISONS COMPRENANT DES DÉRIVÉS DE 6-BENZYLPHÉNYL-2-SULFURTÉRAHYDROPYRAN-3,4,5-TRIOL EN TANT QU'INHIBITEURS DES CO-TRANSPORTEURS 1 ET 2 SODIUM-GLUCOSE DESTINÉES À ÊTRE UTILISÉS CHEZ LES PATIENTS DIABÉTIQUES

(30) Priority: 02.03.2010 US 309592 P
(43) Date of publication of application: 09.01.2013
(73) Proprietor: Lexicon Pharmaceuticals, Inc., The Woodlands, TX 77381 (US)
(72) Inventor: BROWN, Philip Manton, Dallas TX 75205 (US); FREIMAN, Joel Philip, The Woodlands Texas 77381 (US); POWELL, David Reed, Houston Texas 77096 (US)
(74) Representative: Hewson, Timothy John
(86) International application number: PCT/US2011/026591
(87) International publication number: WO 2011/109333

(56) References cited:
- WO-A1-2010/009197
- WO-A2-2008/042688
- Lexicon Pharmaceuticals INc: "Lexicon Pharmaceuticals reports positive results from its LX4211 Phase 2 trial in type 2 diabetic patients", , 20 January 2010 (2010-01-20), XP002632758, Retrieved from the Internet: URL:http://www.news-medical.net/news/20100 120/Lexicon-Pharmaceuticals-reports-positi ve-results-from-its-LX4211-Phase-2-trial-i n-type-2-diabetic-patients.aspx [retrieved on 2011-04-04]
- "Study of LX4211 in subjects with type 2 diabetes mellitus (NCT00962065)", , 14 September 2009 (2009-09-14), XP002632802, Retrieved from the Internet: URL:https://integrity.thomson-pharma.com/i ntegrity/xmlxsl/pk_ref_list.xml_related_re f_to?p_id=636286&p_origen=PRO&p_tsearch=li nk [retrieved on 2011-04-14]
- KOMOROSKI B ET AL: "Dapagliflozin, a novel, selective SGLT2 inhibitor, improved glycemic control over 2 weeks in patients with type 2 diabetes mellitus.", CLINICAL PHARMACOLOGY AND THERAPEUTICS MAY 2009 LNKD- PUBMED:19129749, vol. 85, no. 5, May 2009 (2009-05), pages 513-519, XP009146835, ISSN: 1532-6535
- Freiman et al.: "LX4211, an SGLT2 Inhibitor, Shows Rapid and Significant Improvement in Glycemic Control over 4 Weeks inPatients with Type 2 Diabetes Mellitus.", , 22 June 2010 (2010-06-22), XP002632759, Retrieved from the Internet: URL:http://www.endojournals.org/abstracts/ abstracts.dtl [retrieved on 2011-04-04]
- Freiman et al.: "LX4211, an SGLT2 Inhibitor, Shows Improvements in Cardiovascular Risk Factors over 4 Weeks in Patients with Type 2 Diabetes Mellitus.", , 21 June 2010 (2010-06-21), XP002632760, Retrieved from the Internet: URL:http://www.posters2view.com/endo/ [retrieved on 2011-04-04]
- FREIMAN J ET AL: "LX4211, a dual SGLT2/SGLT1 inhibitor, shows rapid and significant improvement in glycemic control over 28 days in patients with type 2 diabetes (T2DM)", DIABETES, AMERICAN DIABETES ASSOCIATION, US, vol. 59, no. suppl. 1, 25 June 2010 (2010-06-25), pages LB5-LB6, XP009146827, ISSN: 0012-1797
- "Study of LX4211 in subjects with type 2 diabetes mellitus", , 4 February 2011 (2011-02-04), XP002632803, Retrieved from the Internet: URL:http://clinicaltrials.gov/ct2/show/res ults/NCT00962065?sect=X0125all [retrieved on 2011-04-14]
- WASHBURN WILLIAM N: "Evolution of sodium glucose co-transporter 2 inhibitors as anti-diabetic agents", EXPERT OPINION ON THERAPEUTIC PATENTS, INFORMA HEALTHCARE, GB, vol. 19, no. 11, 1 November 2009 (2009-11-01), pages 1485-1499, XP002593697, ISSN: 1354-3776, DOI: DOI:10.1517/13543770903337828
- Lexicon Pharmaceuticals: "LX4211", , 4 April 2011 (2011-04-04), XP002632761, Retrieved from the Internet: URL:http://www.lexicon-genetics.com/pipeli ne/lx4211.html [retrieved on 2011-04-04]
- John Doupis ET AL: "DPP4 Inhibitors: a new approach in diabetes treatment", Advances in Therapy, vol. 25, no. 7, 1 July 2008 (2008-07-01), pages 627-643, XP55071538, ISSN: 0741-238X, DOI: 10.1007/s12325-008-0076-1

## Description

### 1. FIELD OF THE INVENTION

This invention relates to combinations for use in improving the cardiovascular and/or metabolic health of patients, particularly those suffering from type 2 diabetes.

### 2. BACKGROUND

Type 2 diabetes mellitus (T2DM) is a disorder characterized by elevated serum glucose. One way of reducing serum glucose in patients suffering from the disease is by inhibiting glucose reabsorption in the kidney. The kidney plays an important role in the overall control of glucose, since glucose is filtered through the glomeruli at the rate of approximately 8 g/h and is almost completely reabsorbed in the proximal tubule via sodium-glucose cotransporters (SGLTs). Komoroski, B., et al., Clin Pharmacol Ther. 85(5):513-9 (2009). Sodium-glucose cotransporter 2 (SGLT2) is one of 14 transmembrane-domain SGLTs, and is responsible for reabsorbing most of the glucose filtered at the glomerulus. Thus, inhibition of SGLT2 is a rational approach to treating T2DM. *Id.*

A large number of SGLT2 inhibitors have been reported. *See, e.g.,* U.S. patent nos. 6,414,126; 6,555,519; and 7,393,836. One of them, dapagliflozin, has been administered to T2DM patients with promising results. In particular, patients randomized to the compound in a 14-day study exhibited reduced fasting plasma levels and improved glucose tolerance compared to placebo. Komoroski at 513. In a 12-week study, patients randomized to the compound exhibited an improvement in hemoglobin A1c, some weight loss, and some improvement in systolic blood pressure compared to placebo. List, J.F., et al., Diabetes Care. 32(4):650-7 (2009).

Most pharmaceutical efforts directed at discovering and developing inhibitors of SGLT2 "have focused on devising inhibitors selective for the SGLT2 transporter." Washburn, W.N., Expert Opin. Ther. Patents 19(11):1485,1499, 1486 (2009). This is apparently based, at least in part, on the fact that while humans lacking a functional SGLT2 gene appear to live normal lives-apart from exhibiting high urinary glucose excretion-those bearing a SGLT1 gene mutation experience glucose-galactose malsorption. *Id.* Unlike SGLT2, which is expressed exclusively in the human kidney, SGLT1 is also expressed in the small intestine and heart. *Id.*

### 3. SUMMARY OF THE INVENTION

This invention relates, in part, to a dose of 300mg/day or less of a dual SGLT1/2 inhibitor of the formula (I): 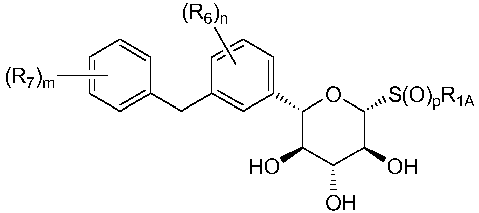 or a pharmaceutically acceptable salt thereof, for use in improving the cardio vascular or metabolic health of a diabetic or prediabetic patient currently taking a DPP-4 inhibitor, wherein: each R_{1A} is independently hydrogen, alkyl, aryl or heterocycle; each R₆ is independently hydrogen, hydroxyl, amino, alkyl, aryl, cyano, halogen, heteroalkyl, heterocycle, nitro, C≡CR_{6A}, OR_{6A}, SR_{6A}, SOR_{6A}, SO₂R_{6A}, C(O)R_{6A}, CO₂R_{6A}, CO₂H, CON(R_{6A})(R_{6A}), CONH(R_{6A}), CONH₂, NHC(O)R_{6A}, or NHSO₂R_{6A}; each R_{6A} is independently alkyl, aryl or heterocycle; each R₇ is independently hydrogen, hydroxyl, amino, alkyl, aryl, cyano, halogen, heteroalkyl, heterocycle, nitro, C≡CR_{7A}, OR_{7A}, SR_{7A}, SOR_{7A}, SO₂R_{7A}, C(O)R_{7A}, CO₂R_{7A}, CO₂H, CON(R_{7A})(R_{7A}), CONH(R_{7A}), CONH₂, NHC(O)R_{7A}, or NHSO₂R_{7A}; each R_{7A} is independently alkyl, aryl or heterocycle; m is 1-4; n is 1-3; and p is 0-2; wherein each alkyl, aryl, heteroalkyl or heterocycle is optionally substituted with one or more of alkoxy, amino, cyano, halo, hydroxyl, or nitro, and wherein the DPP-4 inhibitor is sitagliptin.

In one embodiment of the invention, the administration effects a decrease in the patient's plasma glucose. In one embodiment, the administration effects an improved oral glucose tolerance in the patient. In one embodiment, the administration lowers the patient's post-prandial plasma glucose level. In one embodiment, the administration lowers the patient's plasma fructosamine level. In one embodiment, the administration lowers the patient's HbA1c level. In one embodiment, the administration reduces the patient's blood pressure (*e.g.,* systolic and diastolic). In one embodiment, the administration reduces the patient's triglyceride levels.

The safe and efficacious amount is 300 mg/day or less (e.g., 250, 200, 150, 100, or 50 mg/day or less).

### 4. BRIEF DESCRIPTION OF THE FIGURES

Certain aspects of this disclosure may be understood with reference to the figures, which provide results obtained from a randomized, double-blind, placebo controlled Phase 2a clinical trial, wherein 150 mg and 300 mg doses of a compound of formula (I) were orally administered once daily to patients with type 2 diabetes mellitus.
FIG. 1 shows the plasma glucose levels of patients in the placebo group and in the 150 mg/day and 300 mg/day treatment groups over the course of the study.
FIG. 2 shows each group's mean results in a glucose tolerance test administered over the course of the study.
FIG. 3 shows each group's mean glucose plasma level area under the curve (AUC) over the course of the study.
FIG. 4 shows the results of each group's mean homeostatic model assessment (HOMA) value. Measurements were obtained before the study began and again on day 27.
FIG. 5 provides measurements of each group's mean post-prandial glucose level over the course of the study.
FIG. 6 provides measurements of each group's mean plasma fructosamine level over the course of the study.
FIG. 7 provides each group's mean percent change in hemoglobin A1c level over the course of the study.
FIG. 8 shows the change in each group's mean diastolic blood pressure as measured on day 28 of the study compared to baseline.
FIG. 9 shows the change in each group's mean systolic blood pressure as measured on day 28 of the study compared to baseline.
FIG. 10 shows the change in each group's mean arterial pressure as measured on day 28 of the study compared to baseline.

### 5. DETAILED DESCRIPTION

This disclosure is based, in part, on findings-provided herein and partially disclosed in a press release on 20.01.2010 "Lexicon Pharmaceuticals reports positive results to its LX4211 Phase 2 trial in type 2 diabetic patients"-obtained , from a randomized, double-blind, placebo controlled Phase 2a clinical trial, wherein 150 mg/day and 300 mg/day doses of a dual SGLT1/2 inhibitor were administered to patients with type 2 diabetes mellitus. The dual SGLT1/2 inhibitor was (2S,3R,4R,5S,6R)-2-(4-chloro-3-(4-ethoxybenzyl)phenyl)-6-(methylthio)tetrahydro-2H-pyran-3,4,5-triol, which has the structure:

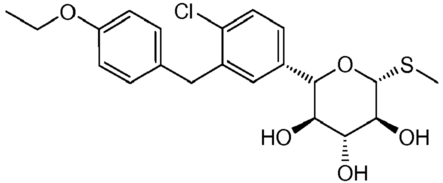

The surprising nature of the clinical study findings-particularly as compared to results reported for selective SGLT2 inhibitors such as dapagliflozin-may be attributed to the compound's ability to inhibit both SGLT2 and SGLT1. Inhibition of SGLT1 has been linked to an increase in glucagon-like peptide-1 (GLP-1) levels. *See, e.g.,* Moriya, R., et al., Am J Physiol Endocrinol Metab 297: E1358-E1365 (2009). Increased GLP-1 levels are known benefit diabetic patients, and a number of well-known diabetes drugs, including sitagliptin, vildagliptin, and saxagliptin, work by inhibiting the enzyme (DPP-4) responsible for GLP-1 degradation.

### 5.1. Definitions

Unless otherwise indicated, the term "alkenyl" means a straight chain, branched and/or cyclic hydrocarbon having from 2 to 20 (e.g., 2 to 10 or 2 to 6) carbon atoms, and including at least one carbon-carbon double bond. Representative alkenyl moieties include vinyl, allyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, 1-hexenyl, 2-hexenyl, 3-hexenyl, 1-heptenyl, 2-heptenyl, 3-heptenyl, 1-octenyl, 2-octenyl, 3-octenyl, 1-nonenyl, 2-nonenyl, 3-nonenyl, 1-decenyl, 2-decenyl and 3-decenyl.

Unless otherwise indicated, the term "alkoxy" means an -0-alkyl group. Examples of alkoxy groups include, but are not limited to, -OCH₃, -OCH₂CH₃, -O(CH₂)₂CH₃, -O(CH₂)₃CH₃,-O(CH₂)₄CH₃, and -O(CH₂)₅CH₃.

Unless otherwise indicated, the term "alkyl" means a straight chain, branched and/or cyclic ("cycloalkyl") hydrocarbon having from 1 to 20 (*e.g.,* 1 to 10 or 1 to 4) carbon atoms. Alkyl moieties having from 1 to 4 carbons are referred to as "lower alkyl." Examples of alkyl groups include, but are not limited to, methyl, ethyl, propyl, isopropyl, n-butyl, t-butyl, isobutyl, pentyl, hexyl, isohexyl, heptyl, 4,4-dimethylpentyl, °Ctyl, 2,2,4-trimethylpentyl, nonyl, decyl, undecyl and dodecyl. Cycloalkyl moieties may be monocyclic or multicyclic, and examples include cyclopropyl, cyclobutyl, cyclopentyl, cyclohexyl, and adamantyl. Additional examples of alkyl moieties have linear, branched and/or cyclic portions (*e.g.,* 1-ethyl-4-methyl-cyclohexyl). The term "alkyl" includes saturated hydrocarbons as well as alkenyl and alkynyl moieties.

Unless otherwise indicated, the term "alkylaryl" or "alkyl-aryl" means an alkyl moiety bound to an aryl moiety.

Unless otherwise indicated, the term "alkylheteroaryl" or "alkyl-heteroaryl" means an alkyl moiety bound to a heteroaryl moiety.

Unless otherwise indicated, the term "alkylheterocycle" or "alkyl-heterocycle" means an alkyl moiety bound to a heterocycle moiety.

Unless otherwise indicated, the term "alkynyl" means a straight chain, branched or cyclic hydrocarbon having from 2 to 20 (*e.g.,* 2 to 20 or 2 to 6) carbon atoms, and including at least one carbon-carbon triple bond. Representative alkynyl moieties include acetylenyl, propynyl, 1-butynyl, 2-butynyl, 1-pentynyl, 2-pentynyl, 3-methyl-1-butynyl, 4-pentynyl, 1-hexynyl, 2-hexynyl, 5-hexynyl, 1-heptynyl, 2-heptynyl, 6-heptynyl, 1-octynyl, 2-octynyl, 7-octynyl, 1-nonynyl, 2-nonynyl, 8-nonynyl, 1-decynyl, 2-decynyl and 9-decynyl.

Unless otherwise indicated, the term "aryl" means an aromatic ring or an aromatic or partially aromatic ring system composed of carbon and hydrogen atoms. An aryl moiety may comprise multiple rings bound or fused together. Examples of aryl moieties include, but are not limited to, anthracenyl, azulenyl, biphenyl, fluorenyl, indan, indenyl, naphthyl, phenanthrenyl, phenyl, 1,2,3,4-tetrahydro-naphthalene, and tolyl.

Unless otherwise indicated, the term "arylalkyl" or "aryl-alkyl" means an aryl moiety bound to an alkyl moiety.

Unless otherwise indicated, the term "dual SGLT1/2 inhibitor" refers to a compound having a ratio of SGLT1 IC₅₀ to SGLT2 IC₅₀ of less than about 75, 50, or 25.

Unless otherwise indicated, the terms "halogen" and "halo" encompass fluorine, chlorine, bromine, and iodine.

Unless otherwise indicated, the term "heteroalkyl" refers to an alkyl moiety (*e.g.,* linear, branched or cyclic) in which at least one of its carbon atoms has been replaced with a heteroatom (*e.g.,* N, O or S).

Unless otherwise indicated, the term "heteroaryl" means an aryl moiety wherein at least one of its carbon atoms has been replaced with a heteroatom (*e.g.,* N, O or S). Examples include, but are not limited to, acridinyl, benzimidazolyl, benzofuranyl, benzoisothiazolyl, benzoisoxazolyl, benzoquinazolinyl, benzothiazolyl, benzoxazolyl, furyl, imidazolyl, indolyl, isothiazolyl, isoxazolyl, oxadiazolyl, oxazolyl, phthalazinyl, pyrazinyl, pyrazolyl, pyridazinyl, pyridyl, pyrimidinyl, pyrimidyl, pyrrolyl, quinazolinyl, quinolinyl, tetrazolyl, thiazolyl, and triazinyl.

Unless otherwise indicated, the term "heteroarylalkyl" or "heteroaryl-alkyl" means a heteroaryl moiety bound to an alkyl moiety.

Unless otherwise indicated, the term "heterocycle" refers to an aromatic, partially aromatic or non-aromatic monocyclic or polycyclic ring or ring system comprised of carbon, hydrogen and at least one heteroatom (*e.g.,* N, O or S). A heterocycle may comprise multiple (*i.e.,* two or more) rings fused or bound together. Heterocycles include heteroaryls. Examples include, but are not limited to, benzo[1,3]dioxolyl, 2,3-dihydro-benzo[1,4]dioxinyl, cinnolinyl, furanyl, hydantoinyl, morpholinyl, oxetanyl, oxiranyl, piperazinyl, piperidinyl, pyrrolidinonyl, pyrrolidinyl, tetrahydrofuranyl, tetrahydropyranyl, tetrahydropyridinyl, tetrahydropyrimidinyl, tetrahydrothiophenyl, tetrahydrothiopyranyl and valerolactamyl.

Unless otherwise indicated, the term "heterocyclealkyl" or "heterocycle-alkyl" refers to a heterocycle moiety bound to an alkyl moiety.

Unless otherwise indicated, the term "heterocycloalkyl" refers to a non-aromatic heterocycle.

Unless otherwise indicated, the term "heterocycloalkylalkyl" or "heterocycloalkyl-alkyl" refers to a heterocycloalkyl moiety bound to an alkyl moiety.

Unless otherwise indicated, the terms "manage," "managing" and "management" encompass preventing the recurrence of the specified disease or disorder in a patient who has already suffered from the disease or disorder, and/or lengthening the time that a patient who has suffered from the disease or disorder remains in remission. The terms encompass modulating the threshold, development and/or duration of the disease or disorder, or changing the way that a patient responds to the disease or disorder.

Unless otherwise indicated, the term "pharmaceutically acceptable salts" refers to salts prepared from pharmaceutically acceptable non-toxic acids or bases including inorganic acids and bases and organic acids and bases. Suitable pharmaceutically acceptable base addition salts include, but are not limited to, metallic salts made from aluminum, calcium, lithium, magnesium, potassium, sodium and zinc or organic salts made from lysine, N,N'-dibenzylethylenediamine, chloroprocaine, choline, diethanolamine, ethylenediamine, meglumine (N-methylglucamine) and procaine. Suitable non-toxic acids include, but are not limited to, inorganic and organic acids such as acetic, alginic, anthranilic, benzenesulfonic, benzoic, camphorsulfonic, citric, ethenesulfonic, formic, fumaric, furoic, galacturonic, gluconic, glucuronic, glutamic, glycolic, hydrobromic, hydrochloric, isethionic, lactic, maleic, malic, mandelic, methanesulfonic, mucic, nitric, pamoic, pantothenic, phenylacetic, phosphoric, propionic, salicylic, stearic, succinic, sulfanilic, sulfuric, tartaric acid, and p-toluenesulfonic acid. Specific non-toxic acids include hydrochloric, hydrobromic, phosphoric, sulfuric, and methanesulfonic acids. Examples of specific salts thus include hydrochloride and mesylate salts. Others are well-known in the art. *See, e.g.,* Remington's Pharmaceutical Sciences, 18th ed. (Mack Publishing, Easton PA: 1990) and Remington: The Science and Practice of Pharmacy, 19th ed. (Mack Publishing, Easton PA: 1995).

Unless otherwise indicated, the terms "prevent," "preventing" and "prevention" contemplate an action that occurs before a patient begins to suffer from the specified disease or disorder, which inhibits or reduces the severity of the disease or disorder. In other words, the terms encompass prophylaxis.

Unless otherwise indicated, a "prophylactically effective amount" of a compound is an amount sufficient to prevent a disease or condition, or one or more symptoms associated with the disease or condition, or prevent its recurrence. A "prophylactically effective amount" of a compound means an amount of therapeutic agent, alone or in combination with other agents, which provides a prophylactic benefit in the prevention of the disease. The term "prophylactically effective amount" can encompass an amount that improves overall prophylaxis or enhances the prophylactic efficacy of another prophylactic agent.

Unless otherwise indicated, the term "SGLT1 IC₅₀" is the IC₅₀ of a compound determined using the *in vitro* human SGLT1 inhibition assay described in the Examples, below.

Unless otherwise indicated, the term "SGLT2 IC₅₀" is the IC₅₀ of a compound determined using the *in vitro* human SGLT2 inhibition assay described in the Examples, below.

Unless otherwise indicated, the term "substituted," when used to describe a chemical structure or moiety, refers to a derivative of that structure or moiety wherein one or more of its hydrogen atoms is substituted with an atom, chemical moiety or functional group such as, but not limited to, alcohol, aldehylde, alkoxy, alkanoyloxy, alkoxycarbonyl, alkenyl, alkyl (*e.g.,* methyl, ethyl, propyl, t-butyl), alkynyl, alkylcarbonyloxy (-OC(O)alkyl), amide (-C(O)NH-alkyl- or-alkylNHC(O)alkyl), amidinyl (-C(NH)NH-alkyl or -C(NR)NH₂), amine (primary, secondary and tertiary such as alkylamino, arylamino, arylalkylamino), aroyl, aryl, aryloxy, azo, carbamoyl (-NHC(O)O-alkyl- or -OC(O)NH-alkyl), carbamyl (*e.g.,* CONH₂, as well as CONH-alkyl, CONH-aryl, and CONH-arylalkyl), carbonyl, carboxyl, carboxylic acid, carboxylic acid anhydride, carboxylic acid chloride, cyano, ester, epoxide, ether (*e.g.,* methoxy, ethoxy), guanidino, halo, haloalkyl (*e.g.,* -CCl₃, -CF₃, -C(CF₃)₃), heteroalkyl, hemiacetal, imine (primary and secondary), isocyanate, isothiocyanate, ketone, nitrile, nitro, oxygen (*i.e.,* to provide an oxo group), phosphodiester, sulfide, sulfonamido (*e.g.,* SO₂NH₂), sulfone, sulfonyl (including alkylsulfonyl, arylsulfonyl and arylalkylsulfonyl), sulfoxide, thiol (*e.g.,* sulfhydryl, thioether) and urea (-NHCONH-alkyl-). In a particular embodiment, the term substituted refers to a derivative of that structure or moiety wherein one or more of its hydrogen atoms is substituted with alcohol, alkoxy, alkyl (*e.g.,* methyl, ethyl, propyl, t-butyl), amide (-C(O)NH-alkyl- or -alkylNHC(O)alkyl), amidinyl (-C(NH)NH-alkyl or -C(NR)NH₂), amine (primary, secondary and tertiary such as alkylamino, arylamino, arylalkylamino), aryl, carbamoyl (-NHC(O)O-alkyl- or -OC(O)NH-alkyl), carbamyl (*e.g.,* CONH₂, as well as CONH-alkyl, CONH-aryl, and CONH-arylalkyl), halo, haloalkyl (*e.g.,* -CCl₃, -CF₃, -C(CF₃)₃), heteroalkyl, imine (primary and secondary), isocyanate, isothiocyanate, thiol (*e.g.,* sulfhydryl, thioether) or urea (-NHCONH-alkyl-).

Unless otherwise indicated, a "therapeutically effective amount" of a compound is an amount sufficient to provide a therapeutic benefit in the treatment or management of a disease or condition, or to delay or minimize one or more symptoms associated with the disease or condition. A "therapeutically effective amount" of a compound means an amount of therapeutic agent, alone or in combination with other therapies, which provides a therapeutic benefit in the treatment or management of the disease or condition. The term "therapeutically effective amount" can encompass an amount that improves overall therapy, reduces or avoids symptoms or causes of a disease or condition, or enhances the therapeutic efficacy of another therapeutic agent.

Unless otherwise indicated, the terms "treat," "treating" and "treatment" contemplate an action that occurs while a patient is suffering from the specified disease or disorder, which reduces the severity of the disease or disorder, or retards or slows the progression of the disease or disorder.

Unless otherwise indicated, the term "include" has the same meaning as "include, but are not limited to," and the term "includes" has the same meaning as "includes, but is not limited to." Similarly, the term "such as" has the same meaning as the term "such as, but not limited to."

Unless otherwise indicated, one or more adjectives immediately preceding a series of nouns is to be construed as applying to each of the nouns. For example, the phrase "optionally substituted alky, aryl, or heteroaryl" has the same meaning as "optionally substituted alky, optionally substituted aryl, or optionally substituted heteroaryl."

It should be noted that a chemical moiety that forms part of a larger compound may be described herein using a name commonly accorded it when it exists as a single molecule or a name commonly accorded its radical. For example, the terms "pyridine" and "pyridyl" are accorded the same meaning when used to describe a moiety attached to other chemical moieties. Thus, the two phrases "XOH, wherein X is pyridyl" and "XOH, wherein X is pyridine" are accorded the same meaning, and encompass the compounds pyridin-2-ol, pyridin-3-ol and pyridin-4-ol.

It should also be noted that if the stereochemistry of a structure or a portion of a structure is not indicated with, for example, bold or dashed lines, the structure or the portion of the structure is to be interpreted as encompassing all stereoisomers of it. Moreover, any atom shown in a drawing with unsatisfied valences is assumed to be attached to enough hydrogen atoms to satisfy the valences. In addition, chemical bonds depicted with one solid line parallel to one dashed line encompass both single and double (*e.g.,* aromatic) bonds, if valences permit.

### 5.2. Compounds

This invention related, in part, to compositions comprising a compound of the following formula (I) 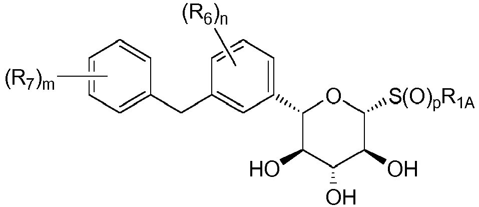 or a pharmaceutically acceptable salt thereof, for use in improving the cardio vascular or metabolic health of a diabetic or prediabetic patient currently taking a DPP-4 inhibitor, wherein: each R_{1A} is independently hydrogen, alkyl, aryl or heterocycle; each R₆ is independently hydrogen, hydroxyl, amino, alkyl, aryl, cyano, halogen, heteroalkyl, heterocycle, nitro, C≡CR_{6A}, OR_{6A}, SR_{6A}, SOR_{6A}, SO₂R_{6A}, C(O)R_{6A}, CO₂R_{6A}, CO₂H, CON(R_{6A})(R_{6A}), CONH(R_{6A}), CONH₂, NHC(O)R_{6A}, or NHSO₂R_{6A}; each R_{6A} is independently alkyl, aryl or heterocycle; each R₇ is independently hydrogen, hydroxyl, amino, alkyl, aryl, cyano, halogen, heteroalkyl, heterocycle, nitro, C≡CR_{7A}, OR_{7A}, SR_{7A}, SOR_{7A}, SO₂R_{7A}, C(O)R_{7A}, CO₂R_{7A}, CO₂H, CON(R_{7A})(R_{7A}), CONH(R_{7A}), CONH₂, NHC(O)R_{7A}, or NHSO₂R_{7A}; each R_{7A} is independently alkyl, aryl or heterocycle; m is 1-4; n is 1-3; and p is 0-2; wherein each alkyl, aryl, heteroalkyl or heterocycle is optionally substituted with one or more of alkoxy, amino, cyano, halo, hydroxyl, or nitro, and wherein the DDP-4 inhibitor is sitagliptin. With regard to the formula (I) disclosed herein, as applicable, particular compounds relating to the invention are such that:
R_{1A} is hydrogen. In others, R_{1A} is optionally substituted alkyl (*e.g.,* optionally substituted lower alkyl).

In some, R₆ is hydrogen, hydroxyl, halogen, OR_{6A} or optionally substituted lower alkyl (*e.g.,* optionally halogenated methyl, ethyl, or isopropyl). In some, R₆ is hydrogen. In some, R₆ is halogen (*e.g.,* chloro). In some, R₆ is hydroxyl. In some, R₆ is OR_{6A} (*e.g.,* methoxy, ethoxy). In some, R₆ is optionally substituted methyl (*e.g.,* CF₃).

In some, R₇ is hydrogen, C≡CR_{7A}, OR_{7A} or optionally substituted lower alkyl (*e.g.,* optionally halogenated methyl, ethyl, or isopropyl). In some, R₇ is hydrogen. In some, R₇ is C≡CR_{7A} and R_{7A} is, for example, optionally substituted (*e.g.,* with lower alkyl or halogen) monocyclic aryl or heterocycle. In some, R₇ is OR_{7A} (*e.g.,* methoxy, ethoxy). In some, R₇ is acetylenyl or optionally substituted methyl or ethyl.

Specific compounds of formula (I) of the invention and also related reference compounds include:
(2S,3R,4R,5S,6R)-2-(4-chloro-3-(4-ethoxybenzyl)phenyl)-6-(methylthio)tetrahydro-2H-pyran-3,4,5-triol;
(2S,3R,4R,5S,6R)-2-(4-chloro-3-(4-ethoxybenzyl)phenyl)-6-(methylsulfonyl)-tetrahydro-2H-pyran-3,4,5-triol;
(2S,3R,4R,5S)-2-[4-Chloro-3-(4-ethoxy-benzyl)-phenyl]-6-methoxy-tetrahydro-pyran-3,4,5-triol;
(3S,4R,5R,6S)-6-[4-Chloro-3-(4-ethoxy-benzyl)-phenyl]-tetrahydro-pyran-2,3,4,5-tetraol;
(2S,3R,4R,5S)-2-[4-Chloro-3-(4-ethoxy-benzyl)-phenyl]-6-ethoxy-tetrahydro-pyran-3,4,5-triol;
(2S,3R,4R,5S,6S)-2-[4-Chloro-3-(4-ethoxy-benzyl)-phenyl]-6-isopropoxy-tetrahydro-pyran-3,4,5-triol;
(2S,3R,4R,5S,6R)-2-[4-Chloro-3-(4-ethoxy-benzyl)-phenyl]-6-isopropoxy-tetrahydro-pyran-3,4,5-triol;
(2S,3R,4R,5S,6R)-2-[4-Chloro-3-(4-ethoxy-benzyl)-phenyl]-6-methoxy-tetrahydro-pyran-3,4,5-triol;
(2S,3R,4R,5S,6S)-2-[4-Chloro-3-(4-ethoxy-benzyl)-phenyl]-6-methoxy-tetrahydro-pyran-3,4,5-triol;
N-{(2S,3S,4R,5R,6S)-6-[4-Chloro-3-(4-ethoxy-benzyl)-phenyl]-3,4,5-trihydroxy-tetrahydro-pyran-2-yl}-N-propyl-acetamide;
(2R,3S,4S,5S)-5-[4-Chloro-3-(4-ethoxy-benzyl)-phenyl]-2,3,4,5-tetrahydroxy-pentanal oxime;
(3S,4R,5R,6S)-6-[4-Chloro-3-(4-ethoxy-benzyl)-phenyl]-3,4,5-trihydroxy-tetrahydro-pyran-2-one oxime;
(2S,3R,4R,5R)-2-[4-Chloro-3-(4-ethoxy-benzyl)-phenyl]-5-fluoro-6-methoxy-tetrahydro-pyran-3,4-diol;
(2S,3R,4R,5S)-2-[4-Chloro-3-(4-hydroxy-benzyl)-phenyl]-6-methoxy-tetrahydro-pyran-3,4,5-triol;
(2S,3R,4S,5R)-2-[4-Chloro-3-(4-ethoxy-benzyl)-phenyl]-tetrahydro-pyran-3,4,5-triol;
(2S,3S,4S,5R)-2-[4-Chloro-3-(4-hydroxy-benzyl)-phenyl]-piperidine-3,4,5-triol;
(2S,3R,4R,5S,6R)-2-[4-Chloro-3-(4-ethoxy-benzyl)-phenyl]-6-ethanesulfinyl-tetrahydro-pyran-3,4,5-triol;
(2S,3R,4R,5S,6R)-2-[4-Chloro-3-(4-ethoxy-benzyl)-phenyl]-6-ethanesulfonyl-tetrahydro-pyran-3,4,5-triol;
Acetic acid (2R,3S,4R,5S,6S)-4,5-diacetoxy-6-[4-chloro-3-(4 ethoxy-benzyl)-phenyl]-2-methylsulfanyl-tetrahydro-pyran-3-yl ester;
(2S,3R,4R,5S,6R)-2-[4-Chloro-3-(4-ethoxy-benzyl)-phenyl]-6-methanesulfonyl-tetrahydro-pyran-3,4,5-triol;
1-{(2S,3S,4S,5R)-2-[4-Chloro-3-(4-ethoxy-benzyl)-phenyl]-3,4,5-trihydroxy-piperidin-1-yl}-ethanon;
(2S,3S,4S,5R)-2-[4-Chloro-3-(4-ethoxy-benzyl)-phenyl]-3,4,5-trihydroxy-piperidine-1-carboxylic acid methyl ester;
(2S,3S,4S,5R)-2-[4-Chloro-3-(4-ethoxy-benzyl)-phenyl]-3,4,5-trihydroxy-piperidine-1-carboxylic acid allyl amide;
(2S,3S,4S,5R)-2-[4-Chloro-3-(4-ethoxy-benzyl)-phenyl]-1-methyl-piperidine-3,4,5-triol;
(2S,3S,4R,5R,6R)-2-[3-(4-Ethoxy-benzyl)-phenyl]-6-hydroxymethyl-1-methyl-piperidine-3,4,5-triol;
(2S,3R,4R,5S,6R)-2-(4-chloro-3-(4-ethoxybenzyl)phenyl)-6-methoxytetrahydro-2H-thiopyran-3,4,5-triol;
(2S,3S,4R,5R,6R)-2-[4-Chloro-3-(4-ethoxy-benzyl)-phenyl]-6-hydroxymethyl-piperidine-3,4,5-triol;
(2S,3S,4R,5R,6R)-2-[4-Chloro-3-(4-ethoxy-benzyl)-phenyl]-6-hydroxymethyl-1-methyl-piperidine-3,4,5-triol;
(2S,3R,4R,5S)-2-[3-(4-Ethoxy-benzyl)-phenyl]-6-methoxy-tetrahydro-pyran-3,4,5-triol;
(2S,3R,4R,5S,6S)-2-[4-Chloro-3-(4-ethoxy-benzyl)-phenyl]-6-(2-hydroxy-ethoxy)-tetrahydro-pyran-3,4,5-triol;
(3S,4R,5R,6S)-2-Benzyloxy-6-[4-chloro-3-(4-ethoxy-benzyl)-phenyl]-tetrahydro-pyran-3,4,5-triol;
(2S,3R,4R,5S)-2-(4'-Ethoxy-biphenyl-3-yl)-6-methoxy-tetrahydro-pyran-3,4,5-triol;
(2S,3R,4R,5S)-2-[4-Chloro-3-(4-ethoxy-benzyl)-phenyl]-6-(2,2,2-trifluoro-ethoxy)-tetrahydro-pyran-3,4,5-triol;
(2S,3R,4R,5S)-2-[4-Chloro-3-(4-ethoxy-benzyl)-phenyl]-6-(2-methoxy-ethoxy)-tetrahydro-pyran-3,4,5-triol
(2S,3R,4R,5S)-2-[4-Chloro-3-(4-ethoxy-benzyl)-phenyl]-6-(2-dimethylamino-ethoxy)-tetrahydro-pyran-3,4,5-triol;
(2S,3R,4R,5S)-2-[4-Chloro-3-(4-ethoxy-benzyl)-phenyl]-6-propylsulfanyl-tetrahydro-pyran-3,4,5-triol;
(2S,3R,4R,5S)-2-[4-Chloro-3-(4-ethoxy-benzyl)-phenyl]-6-imidazol-1-yl-tetrahydro-pyran-3,4,5-triol;
{(3S,4R,5R,6S)-6-[4-Chloro-3-(4-ethoxy-benzyl)-phenyl]-3,4,5-trihydroxy-tetrahydro-pyran-2-yloxy}-acetic acid methyl ester;
(2S,3R,4R,5S)-2-[4-Chloro-3-(4-ethoxy-benzyl)-phenyl]-6-(4-methyl-piperidin-1-yl)-tetrahydro-pyran-3,4,5-triol;
(2S,3R,4R,5S)-2-[4-Chloro-3-(4-ethoxy-benzyl)-phenyl]-6-(5-methyl-thiazol-2-ylamino)-tetrahydropyran-3,4,5-triol;
(2S,3R,4R,5S,6R)-2-[4-Chloro-3-(4-ethoxy-benzyl)-phenyl]-6-phenoxy-tetrahydro-pyran-3,4,5-triol;
N-{(2S,3S,4R,5R,6S)-6-[4-Chloro-3-(4-ethoxy-benzyl)-phenyl]-3,4,5-trihydroxy-tetrahydro-pyran-2-yl}-N-methyl-acetamide;
Acetic acid (2S,3S,4R,5S,6S)-4,5-diacetoxy-6-[4-chloro-3-(4-ethoxy-benzyl)-phenyl]-2-methoxy-tetrahydro-pyran-3-yl ester;
(2S,3R,4R,5S)-2-[4-Chloro-3-(4-ethoxy-phenoxy)-phenyl]-6-methoxy-tetrahydro-pyran-3,4,5-triol;
(2S,3R,4R,5S)-2-[4-Chloro-3-(4-methoxy-phenylsulfanyl)-phenyl]-6-methoxy-tetrahydro-pyran-3,4,5-triol;
(2S,3R,4R,5S)-2-[4-Chloro-3-(4-methoxy-benzenesulfinyl)-phenyl]-6-methoxy-tetrahydro-pyran-3,4,5-triol;
(2S,3R,4R,5S)-2-[4-Chloro-3-(4-ethoxy-benzyl)-phenyl]-6-(3-hydroxy-propoxy)-tetrahydro-pyran-3,4,5-triol;
(2S,3R,4R,5S,6R)-2-[4-Chloro-3-(4-ethoxy-benzyl)-phenyl]-6-(2-hydroxy-ethylsuIfanyl)-tetrahydropyran-3,4,5-triol;
(2S,3R,4R,5S)-2-[4-Chloro-3-(4-ethoxy-benzyl)-phenyl]-6-(2-mercapto-ethoxy)-tetrahydro-pyran-3,4,5-triol;
(2S,3R,4R,5S)-2-[4-Chloro-3-(4-ethoxy-benzyl)-phenyl]-6-(2,3-dihydroxy-propoxy)-tetrahydro-pyran-3,4,5-triol;
(2S,3R,4R,5S)-2-{4-Chloro-3-[4-(2-methoxy-ethoxy)-benzyl]-phenyl}-6-methoxy-tetrahydro-pyran-3,4,5-triol;
(2S,3R,4R,5S,6R)-2-[4-Chloro-3-(4-ethoxy-benzyl)-phenyl]-6-ethylsulfanyl-tetrahydro-pyran-3,4,5-triol;
(2S,3R,4R,5S,6R)-2-[4-Chloro-3-(4-ethoxy-benzyl)-phenyl]-6-methylsulfanyl-tetrahydro-pyran-3,4,5-triol;
[2-Chloro-5-((2S,3R,4R,5S,6S)-3,4,5-trihydroxy-6-methoxy-tetrahydro-pyran-2-yl)-phenyl]-(4-ethoxy-phenyl)-methanone;
(2S,3R,4R,5S,6S)-2-{4-Chloro-3-[(4-ethoxy-phenyl)-hydroxy-methyl]-phenyl}-6-methoxy-tetrahydropyran-3,4,5-triol;
(2S,3R,4R,5S)-2-[3-(4-Ethoxy-benzyl)-4-methyl-phenyl]-6-methoxy-tetrahydro-pyran-3,4,5-triol;
(2S,3R,4R,5S)-2-{4-Chloro-3-[4-(2-methylsulfanyl-ethoxy)-benzyl]-phenyl}-6-methoxy-tetrahydropyran-3,4,5-triol;
(2S,3R,4R,5S)-2-{4-Chloro-3-[4-(pyridin-4-yloxy)-benzyl]-phenyl}-6-methoxy-tetrahydro-pyran-3,4,5-triol;
(2S,3R,4R,5S,6S)-2-(4-Chloro-3-{(4-ethoxy-phenyl)-[(Z)-propylimino]-methyl}-phenyl)-6-methoxy-tetrahydro-pyran-3,4,5-triol;
(2S,3R,4R,5S)-2-{4-Chloro-3-[4-(thiazol-2-yloxy)-benzyl]-phenyl}-6-methoxy-tetrahydro-pyran-3,4,5-triol;
(2S,3R,4R,5S)-2-{4-Chloro-3-[4-(pyrimidin-5-yloxy)-benzyl]-phenyl}-6-methoxy-tetrahydro-pyran-3,4,5-triol;
(2S,3R,4R,5S)-2-{4-Chloro-3-[4-(2,6-dimethoxy-pyrimidin-4-yloxy)-benzyl]-phenyl}-6-methoxy-tetrahydro-pyran-3,4,5-triol;
2-{(2R,3S,4R,5R,6S)-6-[4-Chloro-3-(4-ethoxy-benzyl)-phenyl]-3,4,5-trihydroxy-tetrahydro-pyran-2-ylsulfanyl}-acetamide;
(2S,3R,4R,5S,6R)-2-[4-Chloro-3-(4-ethoxy-benzyl)-phenyl]-6-(furan-2-ylmethylsulfanyl)-tetrahydropyran-3,4,5-triol;
(2S,3R,4R,5S,6S)-2-{4-Chloro-3-[(4-ethoxy-phenyl)-imino-methyl]-phenyl}-6-methoxy-tetrahydropyran-3,4,5-triol;
(2S,3R,4R,5S,6S)-2-{3-[(4-Ethoxy-phenyl)-hydroxy-methyl]-phenyl}-6-methoxy-tetrahydro-pyran-3,4,5-triol;
(2S,3S,4S,5R)-2-[4-Chloro-3-(4-ethoxy-benzyl)-phenyl]-3,4,5-trihydroxy-piperidine-1-carboxylic acid benzyl ester;
(2S,3S,4S,5R)-2-[4-Chloro-3-(4-ethoxy-benzyl)-phenyl]-3,4,5-trihydroxy-piperidine-1-carboxylic acid allylamide;
N-(2-{(2R,3S,4R,5R,6S)-6-[4-Chloro-3-(4-ethoxy-benzyl)-phenyl]-3,4,5-trihydroxy-tetrahydro-pyran-2-ylsulfanyl}-ethyl)-acetamide;
(2S,3R,4R,5S,6R)-2-[4-Chloro-3-(4-ethoxy-benzyl)-phenyl]-6-(2,2,2-trifluoro-ethylsulfanyl)-tetrahydro-pyran-3,4,5-triol;
(2S,3R,4R,5S,6S)-2-{4-Chloro-3-[1-(4-ethoxy-phenyl)-1-hydroxy-ethyl]-phenyl}-6-methoxy-tetrahydro-pyran-3,4,5-triol;
Dimethyl-thiocarbamic acid O-{4-[2-chloro-5-((2S,3R,4R,5S)-3,4,5-trihydroxy-6-methoxy-tetrahydro-pyran-2-yl)-benzyl]-phenyl} ester;
(2S,3R,4R,5S,6S)-2-{3-[1-(4-Ethoxy-phenyl)-ethyl]-phenyl}-6-methoxy-tetrahydro-pyran-3,4,5-triol;
Diethyl-dithiocarbamic acid (2R,3S,4R,5R,6S)-6-[4-chloro-3-(4-ethoxy-benzyl)-phenyl]-3,4,5-trihydroxy-tetrahydro-pyran-2-yl ester;
(2S,3R,4R,5S,6S)-2-(4-Chloro-3-{4-[(R)-(tetrahydro-furan-3-yl)oxy]-benzyl}-phenyl)-6-methoxy-tetrahydro-pyran-3,4,5-triol;
(2S,3R,4R,5S,6R)-2-[4-Chloro-3-(4-ethoxy-benzyl)-phenyl]-6-ethanesulfinyl-tetrahydro-pyran-3,4,5-triol;
(2S,3R,4R,5S)-2-{4-Chloro-3-[4-((S)-1-methyl-pyrrolidin-3-yloxy)-benzyl]-phenyl}-6-methoxy-tetrahydro-pyran-3,4,5-triol;
(2S,3R,4R,5S)-2-{4-Chloro-3-[4-(tetrahydro-pyran-4-yloxy)-benzyl]-phenyl}-6-methoxy-tetrahydropyran-3,4,5-triol;
(2S,3R,4R,5S)-2-(4-Chloro-3-{4-hydroxy-3-[1-(2-methylamino-ethyl)-allyl]-benzyl}-phenyl)-6-methoxy-tetrahydro-pyran-3,4,5-triol;
(2S,3R,4R,5S)-2-{4-Chloro-3-[4-(1-methyl-piperidin-4-yloxy)-benzyl]-phenyl}-6-methoxy-tetrahydropyran-3,4,5-triol;
(2S,3R,4R,5S,6R)-2-[4-Chloro-3-(4-ethoxy-benzyl)-phenyl]-6-methanesulfinyl-tetrahydro-pyran-3,4,5-triol;
(2S,3S,4S,5R)-1-Benzyl-2-[4-chloro-3-(4-ethoxy-benzyl)-phenyl]-piperidine-3,4,5-triol;
(2S,3R,4R,5S)-2-{3-[4-(2-Benzyloxy-ethoxy)-benzyl]-4-chloro-phenyl}-6-methoxy-tetrahydro-pyran-3,4,5-triol;
(2S,3R,4R,5S)-2-{3-[4-(2-Hydroxy-ethoxy)-benzyl]-phenyl}-6-methoxy-tetrahydro-pyran-3,4,5-triol;
(2S,3R,4R,5S)-2-{4-Chloro-3-[4-(2-hydroxy-ethoxy)-benzyl]-phenyl}-6-methoxy-tetrahydro-pyran-3,4,5-triol;
2-{(2S,3S,4S,5R)-2-[4-Chloro-3-(4-ethoxy-benzyl)-phenyl]-3,4,5-trihydroxy-piperidin-1-yl}-acetamide;
(2S,3S,4S,5R)-2-[4-Chloro-3-(4-ethoxy-benzyl)-phenyl]-1-isobutyl-piperidine-3,4,5-triol; (2S,
3R,4R,5S,6R)-2-[4-Chloro-3-(4-ethoxy-benzyl)-phenyl]-6-(2-methyl-tetrahydro-furan-3-ylsulfanyl)-tetrahydro-pyran-3,4,5-triol;
(R)-2-Amino-3-{(2R,3S,4R,5R,6S)-6-[4-chloro-3-(4-ethoxy-benzyl)-phenyl]-3,4,5-trihydroxy-tetrahydro-pyran-2-ylsulfanyl}-propionic acid;
(2S,3R,4R,5S,6R)-2-[4-Chloro-3-(4-ethoxy-benzyl)-phenyl]-6-cyclopentylsulfanyl-tetrahydro-pyran-3,4,5-triol;
(2S,3R,4R,5S,6R)-2-[4-Chloro-3-(4-ethoxy-benzyl)-phenyl]-6-cyclohexylsulfanyl-tetrahydro-pyran-3,4,5-triol;
(2S,3R,4R,5S,6R)-2-[4-Chloro-3-(4-ethoxy-benzyl)-phenyl]-6-(3-methyl-butylsulfanyl)-tetrahydropyran-3,4,5-triol;
(2S,3R,4R,5S)-2-[3-(4-Ethoxy-benzyl)-phenyl]-6-methoxy-tetrahydro-pyran-3,4,5-triol;
1-{(2S,3S,4S,5R)-2-[4-Chloro-3-(4-ethoxy-benzyl)-phenyl]-3,4,5-trihydroxy-piperidin-1-yl}-ethanone;
(2S,3S,4S,5R)-2-[4-Chloro-3-(4-ethoxy-benzyl)-phenyl]-3,4,5-trihydroxy-piperidine-1-carboxylic acid benzyl ester;
(2S,3S,4S,5R)-1-Benzyl-2-[4-chloro-3-(4-ethoxy-benzyl)-phenyl]-piperidine-3,4,5-triol;
2-{(2S,3S,4S,5R)-2-[4-Chloro-3-(4-ethoxy-benzyl)-phenyl]-3,4,5-trihydroxy-piperidin-1-yl}-acetamide;
(2S,3S,4S,5R)-2-[4-Chloro-3-(4-ethoxy-benzyl)-phenyl]-1-isobutyl-piperidine-3,4,5-triol;
(3S,4R,5R)-2-[4-Chloro-3-(4-ethoxy-benzyl)-phenyl]-6-hydroxymethyl-piperidine-3,4,5-triol;
and pharmaceutically acceptable salts thereof.

### 5.3. Methods of Use

This description relates to methods of improving the cardiovascular and/or metabolic health of a patient, which comprise administering to a patient in need thereof a safe and efficacious amount of a dual SGLT1/2 inhibitor (*i.e*. a compound disclosed in Section 5.2 above that is also a dual SGLT1/2 inhibitor).

Patients in need of such improvement include those suffering from, or are at risk of suffering from, type 2 diabetes mellitus.

Optionally, the administration effects a decrease in the patient's fasting plasma glucose level (*e.g.,* by greater than about 50, 55, or 60 mg/dl). The administration may effect an improved oral glucose tolerance in the patient. The administration may lower the patient's post-prandial plasma glucose level. The administration may lower the patient's plasma fructosamine level (*e.g.,* by greater than about 30, 40, or 50 µmol/l). The administration may lower the patient's HbA1c level (*e.g.,* by greater than about 1.0, 1.1, or 1.2 percent) after four weeks of treatment. The administration may reduce the patient's blood pressure (*e.g.,* systolic and diastolic). The administration may reduce the patient's triglyceride levels.

The patient may concurrently be taking another therapeutic agent. Other therapeutic agents include known therapeutic agents useful in the treatment of the aforementioned disorders including: anti-diabetic agents; anti-hyperglycemic agents; hypolipidemic/lipid lowering agents; anti-obesity agents; anti-hypertensive agents and appetite suppressants.

Examples of suitable anti-diabetic agents include biguanides (*e.g.,* metformin, phenformin), glucosidase inhibitors (*e.g.,* acarbose, miglitol), insulins (including insulin secretagogues and insulin sensitizers), meglitinides (*e.g.,* repaglinide), sulfonylureas (*e.g.,* glimepiride, glyburide, gliclazide, chlorpropamide, and glipizide), biguanide/glyburide combinations (*e.g.,* Glucovance), thiazolidinediones (e.g., troglitazone, rosiglitazone, and pioglitazone), PPAR-alpha agonists, PPAR-gamma agonists, PPAR alpha/gamma dual agonists, glycogen phosphorylase inhibitors, inhibitors of fatty acid binding protein (aP2), glucagon-like peptide-1 (GLP-1) or other agonists of the GLP-1 receptor, and dipeptidyl peptidase IV (DPP4) inhibitors.

Examples of meglitinides include nateglinide (Novartis) and KAD1229 (PF/Kissei).

Examples of thiazolidinediones include Mitsubishi's MCC-555 (disclosed in U.S. Pat. No. 5,594,016), Glaxo-Welcome's GL-262570, englitazone (CP-68722, Pfizer), darglitazone (CP-86325, Pfizer, isaglitazone (MIT/J&J), JTT-501 (JPNT/P&U), L-895645 (Merck), R-119702 (Sankyo/WL), NN-2344 (Dr. Reddy/NN), or YM-440 (Yamanouchi).

Examples of PPAR-alpha agonists, PPAR-gamma agonists and PPAR alpha/gamma dual agonists include muraglitizar, peliglitazar, AR-HO39242 (Astra/Zeneca), GW-409544 (Glaxo-Wellcome), GW-501516 (Glaxo-Wellcome), KRP297 (Kyorin Merck) as well as those disclosed by Murakami et al, Diabetes 47, 1841-1847 (1998), WO 01/21602 and in U.S. Pat. No. 6,653,314.

Examples of aP2 inhibitors include those disclosed in U.S. application Ser. No. 09/391,053, filed Sep. 7, 1999, and in U.S. application Ser. No. 09/519,079, filed Mar. 6, 2000, employing dosages as set out herein.

Examples of anti-hyperglycemic agents include glucagon-like peptide-1 (GLP-1), GLP-1(1-36) amide, GLP-1(7-36) amide, GLP-1(7-37) (as disclosed in U.S. Pat. No. 5,614,492), exenatide (Amylin/Lilly), LY-315902 (Lilly), liraglutide (NovoNordisk), ZP-10 (Zealand Pharmaceuticals A/S), CJC-1131 (Conjuchem Inc), and the compounds disclosed in WO 03/033671.

Examples of hypolipidemic/lipid lowering agents include MTP inhibitors, HMG CoA reductase inhibitors, squalene synthetase inhibitors, fibric acid derivatives, ACAT inhibitors, lipoxygenase inhibitors, cholesterol absorption inhibitors, Na⁺/bile acid co-transporter inhibitors, up-regulators of LDL receptor activity, bile acid sequestrants, cholesterol ester transfer protein (*e.g.,* CETP inhibitors, such as CP-529414 (Pfizer) and JTT-705 (Akros Pharma)), and nicotinic acid and derivatives thereof.

Examples of MTP inhibitors include those disclosed in U.S. Pat. No. 5,595,872, U.S. Pat. No. 5,739,135, U.S. Pat. No. 5,712,279, U.S. Pat. No. 5,760,246, U.S. Pat. No. 5,827,875, U.S. Pat. No. 5,885,983 and U.S. Pat. No. 5,962,440.

Examples of HMG CoA reductase inhibitors include mevastatin and related compounds, as disclosed in U.S. Pat. No. 3,983,140, lovastatin (mevinolin) and related compounds, as disclosed in U.S. Pat. No. 4,231,938, pravastatin and related compounds, such as disclosed in U.S. Pat. No. 4,346,227, simvastatin and related compounds, as disclosed in U.S. Pat. Nos. 4,448,784 and 4,450,171. Other HMG CoA reductase inhibitors which may be employed herein include, but are not limited to, fluvastatin, disclosed in U.S. Pat. No. 5,354,772, cerivastatin, as disclosed in U.S. Pat. Nos. 5,006,530 and 5,177,080, atorvastatin, as disclosed in U.S. Pat. Nos. 4,681,893, 5,273,995, 5,385,929 and 5,686,104, atavastatin (Nissan/Sankyo's nisvastatin (NK-104)), as disclosed in U.S. Pat. No. 5,011,930, visastatin (Shionogi-Astra/Zeneca (ZD-4522)), as disclosed in U.S. Pat. No. 5,260,440, and related statin compounds disclosed in U.S. Pat. No. 5,753,675, pyrazole analogs of mevalonolactone derivatives, as disclosed in U.S. Pat. No. 4,613,610, indene analogs of mevalonolactone derivatives, as disclosed in PCT application WO 86/03488, 6-[2-(substituted-pyrrol-1-yl)-alkyl)pyran-2-ones and derivatives thereof, as disclosed in U.S. Pat. No. 4,647,576, Searle's SC-45355 (a 3-substituted pentanedioic acid derivative) dichloroacetate, imidazole analogs of mevalonolactone, as disclosed in PCT application WO 86/07054, 3-carboxy-2-hydroxy-propane-phosphonic acid derivatives, as disclosed in French Patent No. 2,596,393, 2,3-disubstituted pyrrole, furan and thiophene derivatives, as disclosed in European Patent Application No. 0221025, naphthyl analogs of mevalonolactone, as disclosed in U.S. Pat. No. 4,686,237, octahydronaphthalenes, such as disclosed in U.S. Pat. No. 4,499,289, keto analogs of mevinolin (lovastatin), as disclosed in European Patent Application No. 0142146 A2, and quinoline and pyridine derivatives, as disclosed in U.S. Pat. Nos. 5,506,219 and 5,691,322.

Examples of hypolipidemic agents include pravastatin, lovastatin, simvastatin, atorvastatin, fluvastatin, cerivastatin, atavastatin, and ZD-4522.

Examples of phosphinic acid compounds useful in inhibiting HMG CoA reductase include those disclosed in GB 2205837.

Examples of squalene synthetase inhibitors include α-phosphono-sulfonates disclosed in U.S. Pat. No. 5,712,396, those disclosed by Biller et al., J. Med. Chem. 1988, Vol. 31, No. 10, pp 1869-1871, including isoprenoid (phosphinyl-methyl)phosphonates, as well as other known squalene synthetase inhibitors, for example, as disclosed in U.S. Pat. Nos. 4,871,721 and 4,924,024 and in Biller, S. A., et al., Current Pharmaceutical Design, 2, 1-40 (1996).

Examples of additional squalene synthetase inhibitors suitable for use herein include the terpenoid pyrophosphates disclosed by P. Ortiz de Montellano et al., J. Med. Chem., 1977, 20, 243-249, the farnesyl diphosphate analog A and presqualene pyrophosphate (PSQ-PP) analogs as disclosed by Corey and Volante, J. Am. Chem. Soc. 1976, 98, 1291-1293, phosphinylphosphonates reported by McClard, R. W. et al., J.A.C.S., 1987, 109, 5544 and cyclopropanes reported by Capson, T. L., PhD dissertation, June, 1987, Dept. Med. Chem. U of Utah, Abstract, Table of Contents, pp 16, 17, 40-43, 48-51, Summary.

Examples of fibric acid derivatives which may be employed in combination the compounds of this invention include fenofibrate, gemfibrozil, clofibrate, bezafibrate, ciprofibrate, clinofibrate and the like, probucol, and related compounds, as disclosed in U.S. Pat. No. 3,674,836, probucol and gemfibrozil being preferred, bile acid sequestrants, such as cholestyramine, colestipol and DEAE-Sephadex (Secholex, Policexide), as well as lipostabil

(Rhone-Poulenc), Eisai E-5050 (an N-substituted ethanolamine derivative), imanixil (HOE-402), tetrahydrolipstatin (THL), istigmastanylphos-phorylcholine (SPC, Roche), aminocyclodextrin (Tanabe Seiyoku), Ajinomoto AJ-814 (azulene derivative), melinamide (Sumitomo), Sandoz 58-035, American Cyanamid CL-277,082 and CL-283,546 (disubstituted urea derivatives), nicotinic acid, acipimox, acifran, neomycin, p-aminosalicylic acid, aspirin, poly(diallylmethylamine) derivatives, such as disclosed in U.S. Pat. No. 4,759,923, quaternary amine poly(diallyldimethylammonium chloride) and ionenes, such as disclosed in U.S. Pat. No. 4,027,009, and other known serum cholesterol lowering agents.

Examples of ACAT inhibitor that may be employed in combination compounds of this invention include those disclosed in Drugs of the Future 24, 9-15 (1999), (Avasimibe); Nicolosi et al., Atherosclerosis (Shannon, Irel). (1998), 137(1), 77-85; Ghiselli, Giancarlo, Cardiovasc. Drug Rev. (1998), 16(1), 16-30; Smith, C., et al., Boorg. Med. Chem. Lett. (1996), 6(1), 47-50; Krause et al., Editor(s): Ruffolo, Robert R., Jr.; Hollinger, Mannfred A., Inflammation: Mediators Pathways (1995), 173-98, Publisher: CRC, Boca Raton, Fla.; Sliskovic et al., Curr. Med. Chem. (1994), 1(3), 204-25; Stout et al., Chemtracts: Org. Chem. (1995), 8(6), 359-62, or TS-962 (Taisho Pharmaceutical Co. Ltd).

Examples of hypolipidemic agents include up-regulators of LD2 receptor activity, such as MD-700 (Taisho Pharmaceutical Co. Ltd) and LY295427 (Eli Lilly).

Examples of cholesterol absorption inhibitors include SCH48461 (Schering-Plough), as well as those disclosed in Atherosclerosis 115, 45-63 (1995) and J. Med. Chem. 41, 973 (1998).

Examples of ileal Na⁺/bile acid co-transporter inhibitors include compounds as disclosed in Drugs of the Future, 24, 425-430 (1999).

Examples of lipoxygenase inhibitors include 15-lipoxygenase (15-LO) inhibitors, such as benzimidazole derivatives, as disclosed in WO 97/12615, 15-LO inhibitors, as disclosed in WO 97/12613, isothiazolones, as disclosed in WO 96/38144, and 15-LO inhibitors, as disclosed by Sendobry et al., Brit. J. Pharmacology (1997) 120, 1199-1206, and Cornicelli et al., Current Pharmaceutical Design, 1999, 5, 11-20.

Examples of suitable anti-hypertensive agents for use in combination with compounds of this invention include beta adrenergic blockers, calcium channel blockers (L-type and T-type; *e.g.,* diltiazem, verapamil, nifedipine, amlodipine and mybefradil), diuretics (e.g., chlorothiazide, hydrochlorothiazide, flumethiazide, hydroflumethiazide, bendroflumethiazide, methylchlorothiazide, trichloromethiazide, polythiazide, benzthiazide, ethacrynic acid tricrynafen, chlorthalidone, furosemide, musolimine, bumetamide, triamtrenene, amiloride, spironolactone), renin inhibitors, ACE inhibitors (*e.g.,* captopril, zofenopril, fosinopril, enalapril, ceranopril, cilazopril, delapril, pentopril, quinapril, ramipril, lisinopril), AT-1 receptor antagonists (*e.g.,* losartan, irbesartan, valsartan), ET receptor antagonists (*e.g.,* sitaxsentan, atrsentan and compounds disclosed in U.S. Pat. Nos. 5,612,359 and 6,043,265), Dual ET/All antagonist (*e.g.,* compounds disclosed in WO 00/01389), neutral endopeptidase (NEP) inhibitors, vasopepsidase inhibitors (dual NEP-ACE inhibitors) (*e.g.,* omapatrilat and gemopatrilat), and nitrates.

Examples anti-obesity agents include beta 3 adrenergic agonists, a lipase inhibitors, serotonin (and dopamine) reuptake inhibitors, thyroid receptor beta drugs, 5HT_{2C} agonists, (such as Arena APD-356); MCHR1 antagonists such as Synaptic SNAP-7941 and Takeda T-226926, melanocortin receptor (MC4R) agonists, melanin-concentrating hormone receptor (MCHR) antagonists (such as Synaptic SNAP-7941 and Takeda T-226926), galanin receptor modulators, orexin antagonists, CCK agonists, NPY1 or NPY5 antagonsist, NPY2 and NPY4 modulators, corticotropin releasing factor agonists, histamine receptor-3 (H3) modulators, 11-beta-HSD-1 inhibitors, adinopectin receptor modulators, monoamine reuptake inhibitors or releasing agents, a ciliary neurotrophic factor (CNTF, such as AXOKINE by Regeneron), BDNF (brain-derived neurotrophic factor), leptin and leptin receptor modulators, cannabinoid-1 receptor antagonists (such as SR-141716 (Sanofi) or SLV-319 (Solvay)), and/or an anorectic agent.

Examples of beta 3 adrenergic agonists include AJ9677 (Takeda/Dainippon), L750355 (Merck), or CP331648 (Pfizer) or other known beta 3 agonists, as disclosed in U.S. Pat. Nos. 5,541,204, 5,770,615, 5,491,134, 5,776,983 and 5,488,064.

Examples of lipase inhibitors include orlistat and ATL-962 (Alizyme).

Examples of serotonin (and dopoamine) reuptake inhibitors (or serotonin receptor agonists) include BVT-933 (Biovitrum), sibutramine, topiramate (Johnson & Johnson) and axokine (Regeneron).

Examples of thyroid receptor beta compounds include thyroid receptor ligands, such as those disclosed in WO97/21993 (U. Cal SF), WO99/00353 (KaroBio) and GB98/284425 (KaroBio).

Examples of monoamine reuptake inhibitors include fenfluramine, dexfenfluramine, fluvoxamine, fluoxetine, paroxetine, sertraline, chlorphentermine, cloforex, clortermine, picilorex, sibutramine, dexamphetamine, phentermine, phenylpropanolamine and mazindol.

Examples of anorectic agents include dexamphetamine, phentermine, phenylpropanolamine, and mazindol.

### 5.4. Pharmaceutical Formulations

This invention relates to pharmaceutical compositions comprising one or more dual SGLT1/2 inhibitor of formula (I), in combination with sitagliptin.

Certain pharmaceutical compositions are single unit dosage forms suitable for oral administration to a patient. Discrete dosage forms suitable for oral administration include tablets (*e.g.,* chewable tablets), caplets, capsules, and liquids (*e.g.,* flavored syrups). Such dosage forms contain predetermined amounts of active ingredients, and may be prepared by methods of pharmacy well known to those skilled in the art. *See, e.g.,* Remington's Pharmaceutical Sciences, 18th ed. (Mack Publishing, Easton PA: 1990).

Typical oral dosage forms are prepared by combining the active ingredient(s) in an intimate admixture with at least one excipient according to conventional pharmaceutical compounding techniques. Because of their ease of administration, tablets and capsules represent the most advantageous oral dosage unit forms. If desired, tablets can be coated by standard aqueous or nonaqueous techniques. Such dosage forms can be prepared by conventional methods of pharmacy. In general, pharmaceutical compositions and dosage forms are prepared by uniformly and intimately admixing the active ingredients with liquid carriers, finely divided solid carriers, or both, and then shaping the product into the desired presentation if necessary. Disintegrants may be incorporated in solid dosage forms to facility rapid dissolution. Lubricants may also be incorporated to facilitate the manufacture of dosage forms (*e.g.,* tablets).

### 6. EXAMPLES

### 6.1. In Vitro Human SGLT2 Inhibition Assay

Human sodium/glucose co-transporter type 2 (SGLT2; accession number P31639; GI:400337) was cloned into pIRESpuro2 vector for mammalian expression (construct: HA-SGLT2-pIRESpuro2).

HEK293 cells were transfected with the human HA-SGLT2-pIRESpuro2 vector and the bulk stable cell line was selected in presence of 0.5 µg/ml of puromycin. Human HA-SGLT2 cells were maintained in DMEM media containing 10% FBS, 1% GPS and 0.5 µg/ml of puromycin.

The HEK293 cells expressing the human HA-SGLT2 were seeded in 384 well plates (30,000 cells/well) in DMEM media containing 10% FBS, 1% GPS and 0.5 µg/ml of puromycin, then incubated overnight at 37 C, 5% CO₂. Cells were then washed with uptake buffer (140 mM NaCl, 2 mM KCI, 1 mM CaCl₂, 1 mM MgCl₂, 10 mM HEPES, 5 mM Tris, 1 mg/ml bovine serum albumin (BSA), pH 7.3). Twenty microliters of uptake buffer with or without testing compounds were added to the cells. Then, 20 microliters of uptake buffer containing ¹⁴C-AMG (100 nCi) were added to the cells. The cell plates were incubated at 37°C, 5% CO₂ for 1-2 hours. After washing the cells with uptake buffer, scintillation fluid was added (40 microliters/well) and ¹⁴C-AMG uptake was measured by counting radioactivity using a scintillation coulter (TopCoulter NXT; Packard Instruments).

### 6.2. In Vitro Human SGLT1 Inhibition Assay

Human sodium/glucose co-transporter type 1 (SGLT1; accession number NP_000334; GI: 4507031) was cloned into pIRESpuro2 vector for mammalian expression (construct: HA-SGLT1-pIRESpuro2).

HEK293 cells were transfected with the human HA-SGLT1-pIRESpuro2 vector and the bulk stable cell line was selected in presence of 0.5 µg/ml of puromycin. Human HA-SGLT1 cells were maintained in DMEM media containing 10% FBS, 1% GPS and 0.5 µg/ml of puromycin.

The HEK293 cells expressing the human HA-SGLT1 were seeded in 384 well plates (30,000 cells/well) in DMEM media containing 10% FBS, 1% GPS and 0.5 µg/ml of puromycin, then incubated overnight at 37 C, 5% CO₂. Cells were then washed with uptake buffer (140 mM NaCl, 2 mM KCI, 1 mM CaCl₂, 1 mM MgCl₂, 10 mM HEPES, 5 mM Tris, 1 mg/ml bovine serum albumin (BSA), pH 7.3). Twenty microliters of uptake buffer with or without testing compounds were added to the cells. Then, 20 microliters of uptake buffer containing ¹⁴C-AMG (100 nCi) were also added to cells. The cell plates were incubated at 37°C, 5% CO₂ for 1-2 hours. After washing the cells with uptake buffer, scintillation fluid was added (40 microliters/well) and ¹⁴C-AMG uptake was measured by counting radioactivity using a scintillation coulter (TopCoulter NXT; Packard Instruments).

### 6.3. Pharmacology of (2S,3R,4R,5S,6R)-2-(4-chloro-3-(4-ethoxybenzyl)phenly)-6-(methylthio)tetrahydro-2H-pyran-3,4,5-triol

Patients (n = 36) with type 2 diabetes mellitus received one of two oral doses of (2S,3R,4R,5S,6R)-2-(4-chloro-3-(4-ethoxybenzyl)phenyl)-6-(methylthio)tetrahydro-2H-pyran-3,4,5-triol, given as 150 mg or 300 mg once daily, or matching placebo, for 28 days. Preliminary data showed significant and sustained glucosuria over the 28-day dosing period for both dose levels when compared to placebo. Adverse events were generally mild and evenly distributed across all dose groups, including placebo, and no evidence of dose-limiting toxicities was observed.

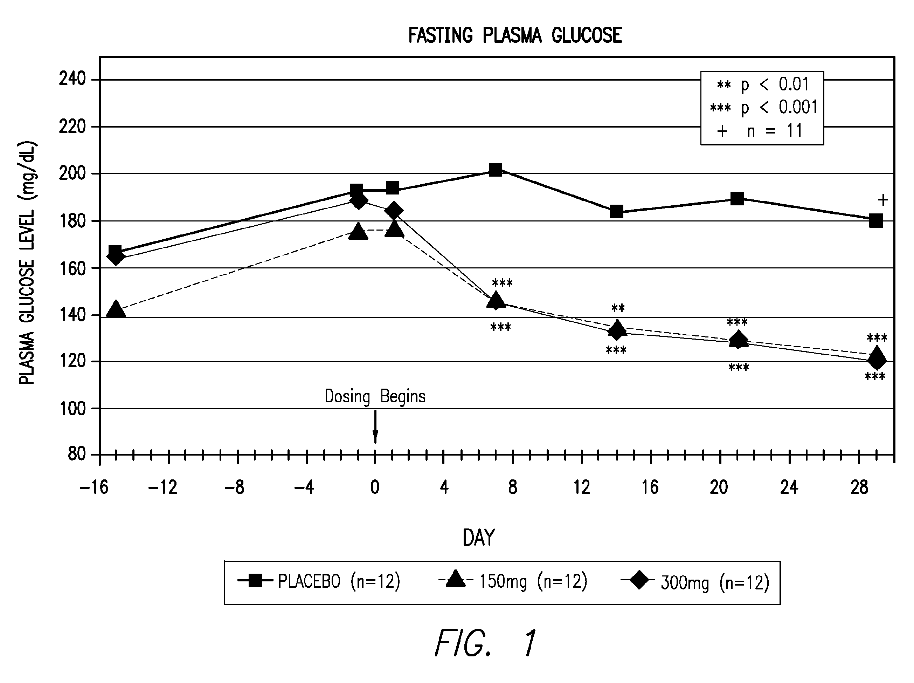

In this study, patients on metformin were taken off of the drug 16 days prior to day 0, the day dosing first began. As shown in FIG. 1, the fasting plasma glucose levels of patients in the placebo group and in the 150 mg/day and 300 mg/day treatment groups increased during that period. Upon treatment, patients in both treatment groups exhibited a rapid, statistically significant decrease in fasting plasma glucose levels, with reductions at week four of 53.4 mg/dl and 65.9 mg/dl in the 150 mg and 300 mg dose groups, respectively, as compared to 15.1 mg/dl for placebo. Notably, a substantial percentage (42%) of patients in the 300 mg dose group achieved fasting plasma glucose levels of <105 mg/dl at week four of dosing as compared to placebo (p=0.037).

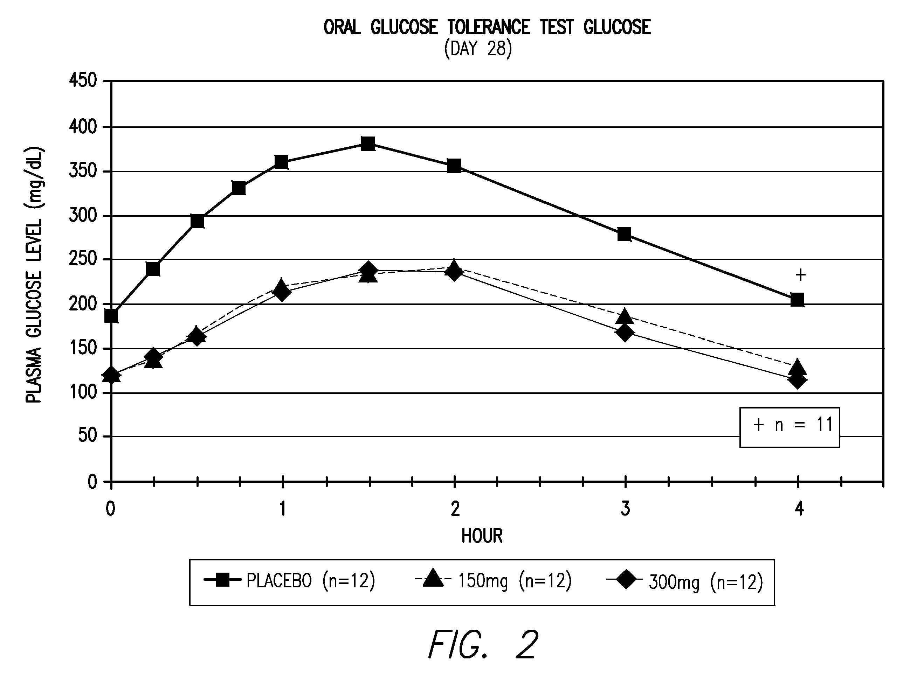

Over the course of the study, the patients' glucose tolerance was tested in a conventional manner. As shown in FIG. 2, patients in both treatment groups exhibited greater glucose tolerance than those in the placebo group.

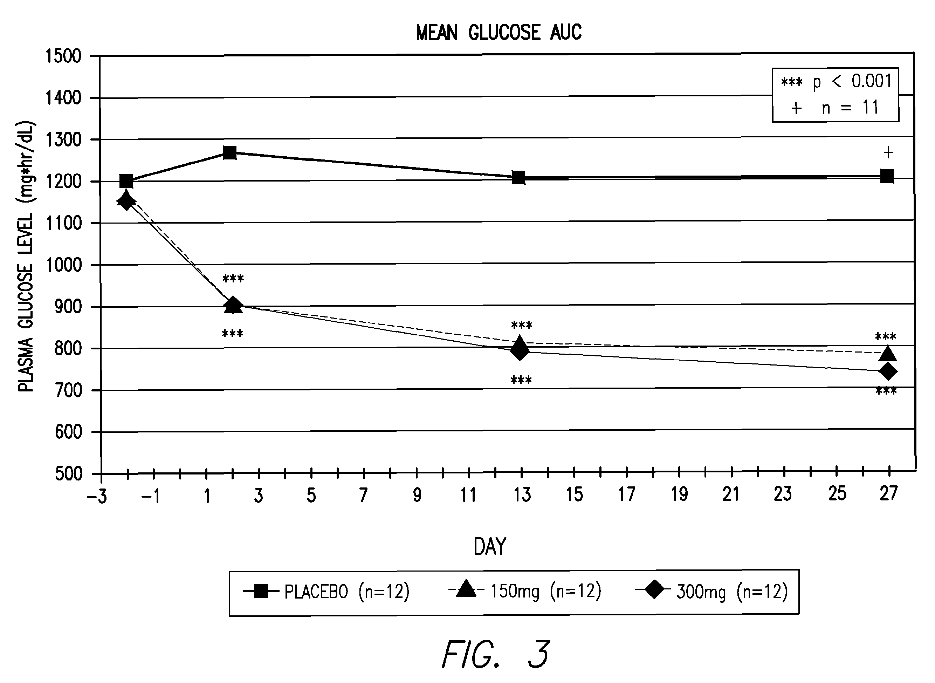

FIG. 3 shows the mean glucose plasma level area under the curve (AUC) of the patients. After just one day of treatment, both the 150 mg/day and 300 mg/day treatment groups exhibited statistically significant decreases in their mean plasma glucose AUCs.

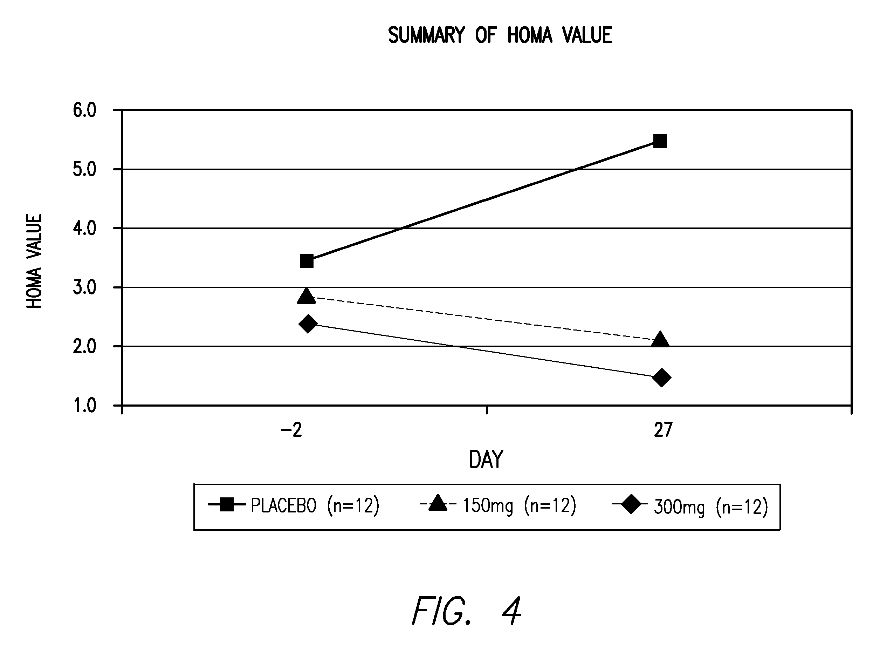

As shown in FIG. 4, patients randomized to the 150 mg/day and 300 mg/day treatment groups showed improved insulin sensitivity compared to placebo. This figure provides a summary of the groups' homeostatic model assessment (HOMA) values.

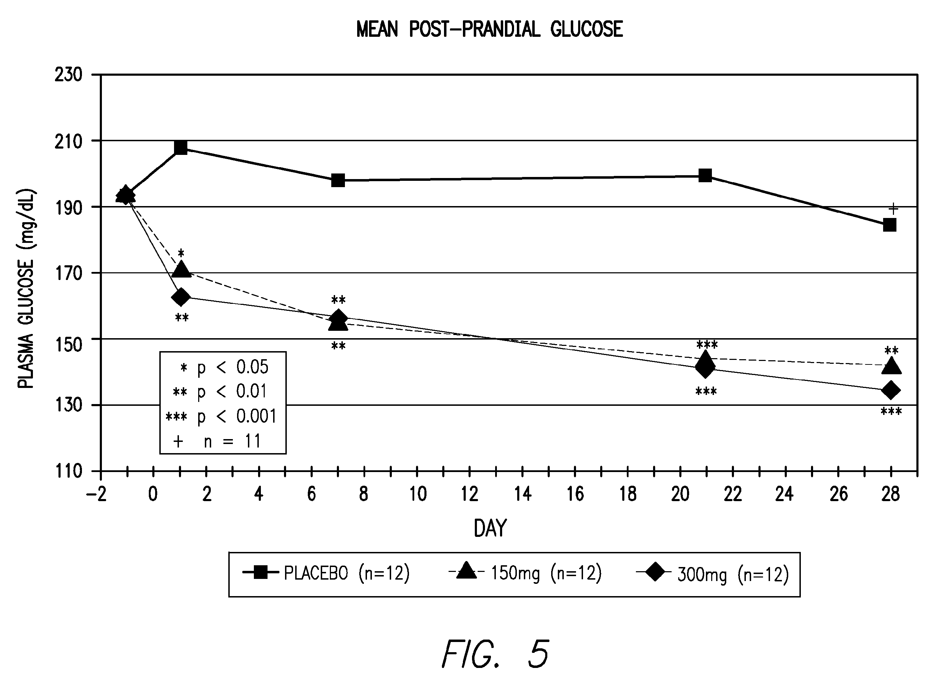

As shown in FIG. 5, patients in both treatment groups exhibited a rapid, statistically significant decrease in post-prandial glucose levels compared to placebo.

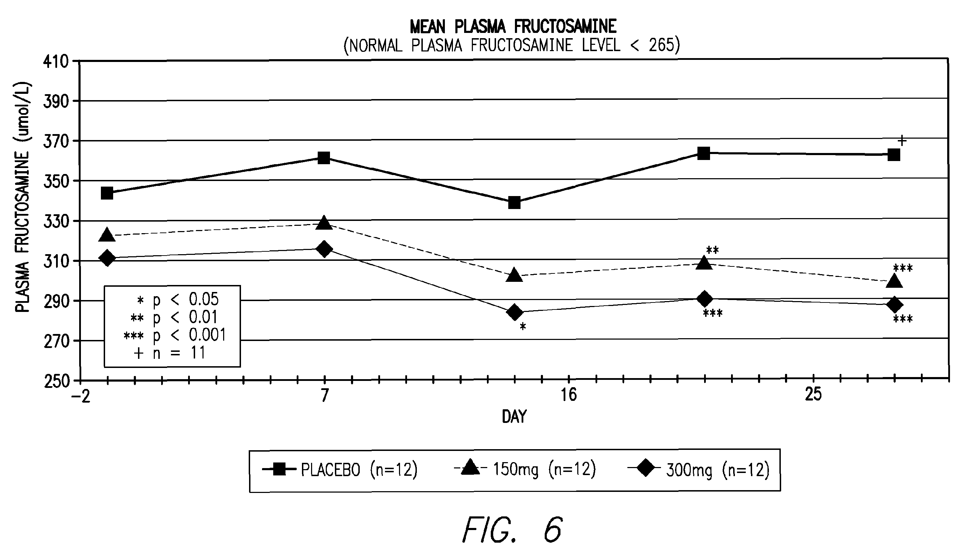

Fructosamine (glycated albumin) is often measured to assess the short-term control of blood sugar. FIG. 6 shows the effect of the compound on patients' mean plasma fructosamine levels.

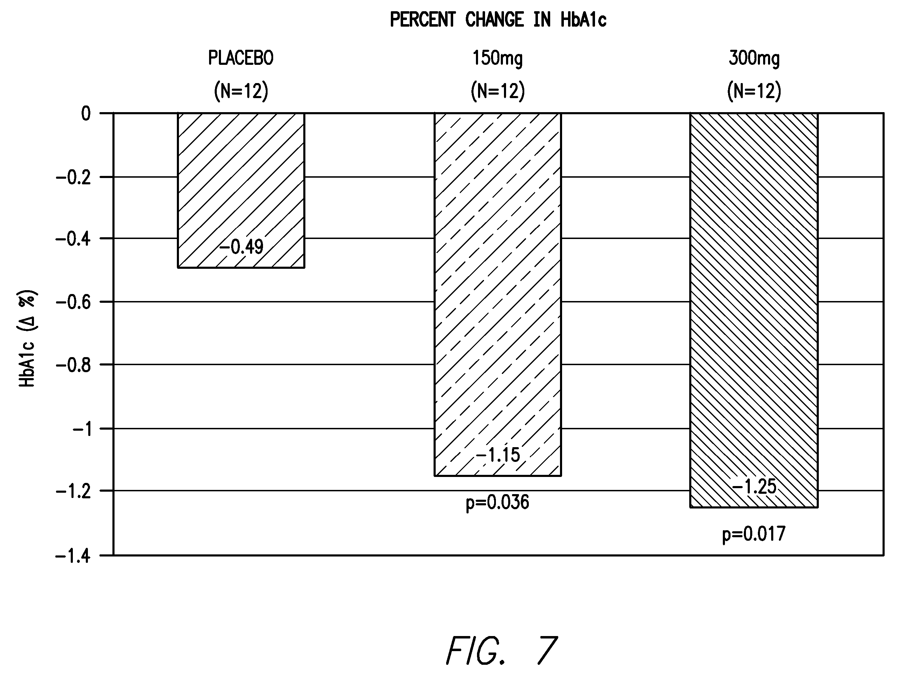

FIG. 7 shows patients' mean percent change in glycated hemoglobin (hemoglobin A1c; HbA1c) levels. HbA1c is a form of hemoglobin used primarily to identify the average plasma glucose concentration over prolonged periods of time. Patients randomized to the 150 mg/day and 300 mg/day treatment groups exhibited a marked decrease in their mean HbA1c levels. After only four weeks of dosing, average percent HbA1c was significantly reduced by 1.15 in the 150 mg dose group (p=0.036) and by 1.25 in the 300 mg dose group (p=0.017), as compared to 0.49 in the placebo group. HbA1c levels were reduced to less than or equal to 7% for half the patients in both dose groups; baseline levels were 8.22%, 8.50% and 8.20% for the 150 mg, 300 mg, and placebo groups, respectively.

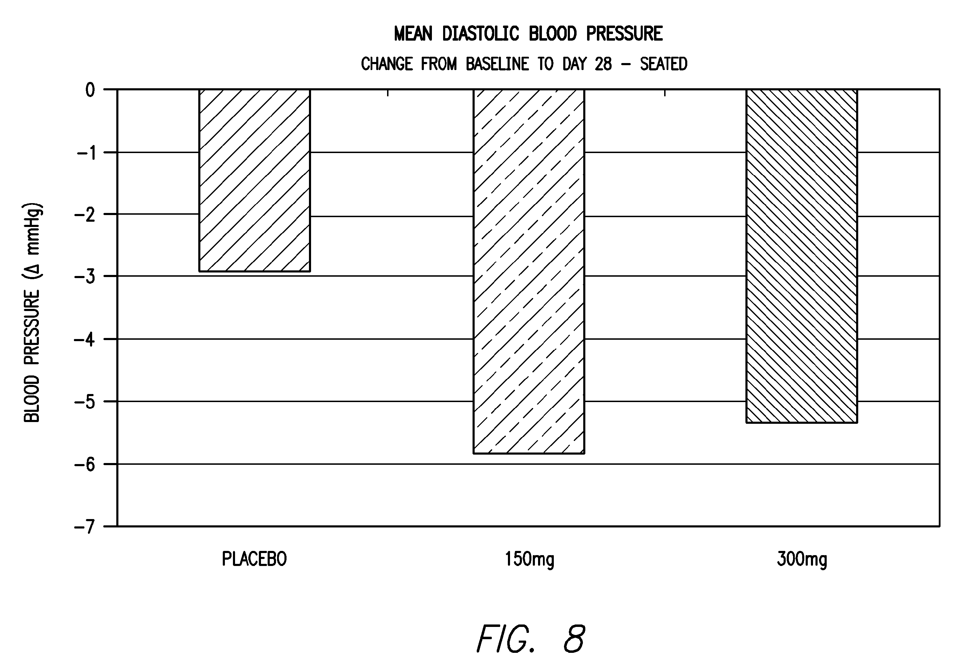

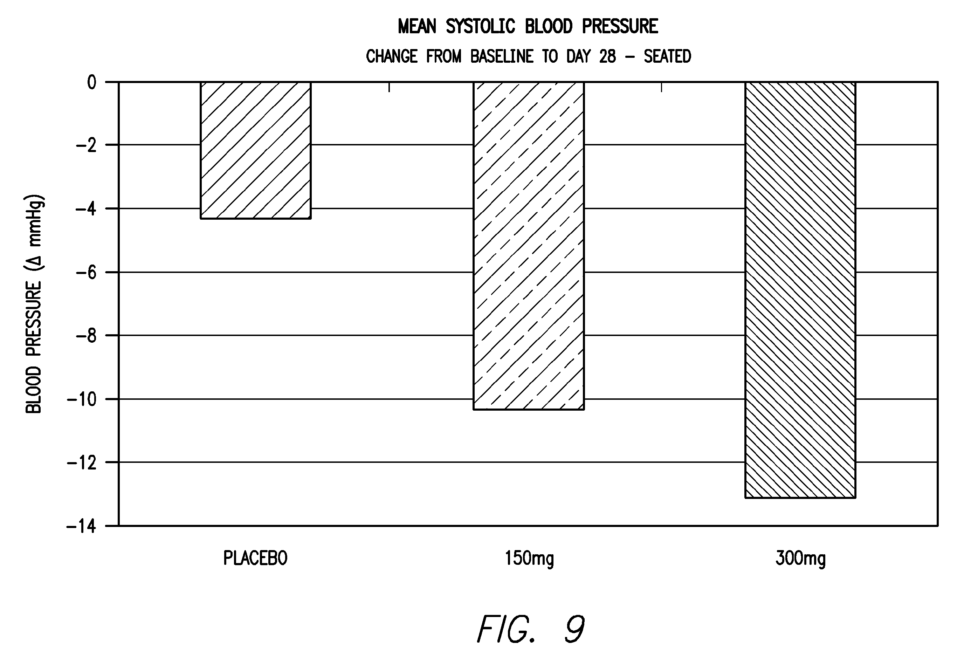

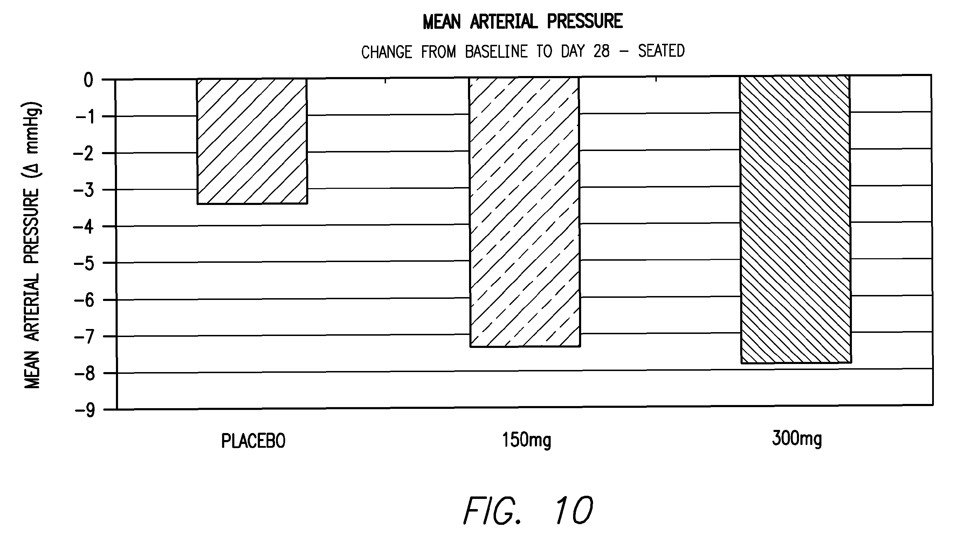

Surprisingly, patients in the 150 mg/day and 300 mg/day treatment groups also exhibited decreased mean diastolic and systolic blood pressures after 28 days of dosing compared to placebo. See FIGS. 8 and 9. And as shown in FIG. 10, the mean arterial pressures of patients in both treatment groups also decreased.

As shown below in Table 1, it was found that administration of the compound also lowered patients' serum triglyceride levels and effected weight loss:

**Table 1**

| **Change from Baseline** | **150 mg (n=12)** | **300 mg (n=12)** | **Placebo (n=12)** |
|---|---|---|---|
| Seated Systolic BP (mmHg) | -10.3 | -13.1 | -4.3 |
| Seated Diastolic BP (mmHg) | -5.8 | -5.3 | -2.9 |
| Serum Triglyceride (mg/dL) | -66.6 | -62.8 | -20.2 |
| Change in Weight (%) | -3.4 | -3.7 | -2.2 |

These results demonstrate that within a four-week treatment period, patients receiving the compound exhibited improvements in blood pressure control, weight reduction, and triglyceride levels that were associated with improvements in glycemic parameters.

## Claims

1. A dose of 300 mg/day or less (e.g., 250, 200, 150, 100, or 50 mg/day or less) of a dual SGLT1/2 inhibitor of the formula: 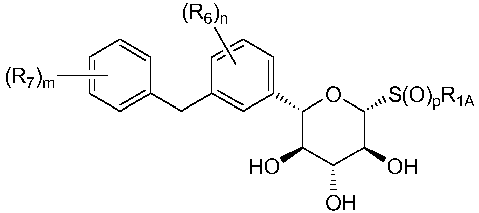 or a pharmaceutically acceptable salt thereof, for use in improving the cardiovascular or metabolic health of a diabetic or pre-diabetic patient currently taking a DPP-4 inhibitor, wherein:
each R_{1A} is independently hydrogen, alkyl, aryl or heterocycle;
each R₆ is independently hydrogen, hydroxyl, amino, alkyl, aryl, cyano, halogen, heteroalkyl, heterocycle, nitro, C≡CR_{6A}, OR_{6A}, SR_{6A}, SOR_{6A}, SO₂R_{6A}, C(O)R_{6A}, CO₂R_{6A}, CO₂H, CON(R_{6A})(R_{6A}), CONH(R_{6A}), CONH₂, NHC(O)R_{6A}, or NHSO₂R_{6A};
each R_{6A} is independently alkyl, aryl or heterocycle;
each R₇ is independently hydrogen, hydroxyl, amino, alkyl, aryl, cyano, halogen, heteroalkyl, heterocycle, nitro, C≡CR_{7A}, OR_{7A}, SR_{7A}, SOR_{7A}, SO₂R_{7A}, C(O)R_{7A}, CO₂R_{7A}, CO₂H, CON(R_{7A})(R_{7A}), CONH(R_{7A}), CONH₂, NHC(O)R_{7A}, or NHSO₂R_{7A};
each R_{7A} is independently alkyl, aryl or heterocycle;
m is 1-4;
n is 1-3;
p is 0-2; and
wherein each alkyl, aryl, heteroalkyl or heterocycle is optionally substituted with one or more of alkoxy, amino, cyano, halo, hydroxyl, or nitro, and wherein the DPP-4 inhibitor is sitagliptin.

2. A dose for use according to claim 1, wherein the improvement is a lowering of the patient's fasting plasma glucose level by greater than about 50, 55, or 60 mg/dl.

3. A dose for use according to claim 1, wherein the improvement is a lowering of the patient's HbA1c level by greater than about 1.0, 1.1, or 1.2 percent

4. A dose for use according to claim 1, wherein the improvement is a lowering of the patient's plasma fructosamine level by greater than about 30, 40, or 50 µmol/l.

5. A dose for use according to claim 1, wherein improvement is a lowering of the patient's blood pressure.

6. A dose for use according to claim 1, wherein the improvement is a lowering of the patient's triglyceride levels.

7. A dose for use according to claim 1, wherein the dual SGLT1/2 inhibitor is of the formula:

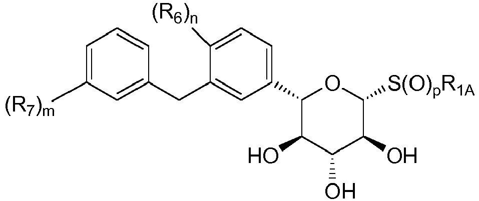

8. A dose for use according to claim 7, wherein the dual SGLT1/2 inhibitor is of the formula:

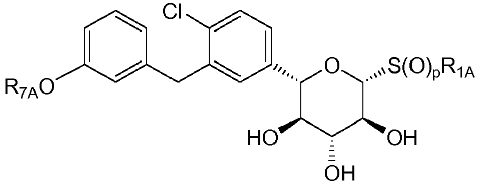

9. A dose for use according to claim 8, wherein the dual SGLT1/2 inhibitor is of the formula:

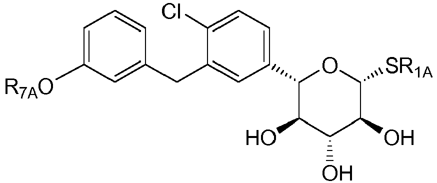

10. A dose for use according to claim 9, wherein R_{7A} is methyl or ethyl.

11. A dose for use according to claim 9, wherein R_{1A} is methyl.

12. A dose for use according to claim 11, wherein the dual SGLT1/2 inhibitor is (2S,3R,4R,5S,6R)-2-(4-chloro-3-(4-ethoxybenzyl)phenyl)-6-(methylthio)tetrahydro-2H-pyran-3,4,5-triol.

## Patentansprüche

1. Dosis von 300 mg oder weniger pro Tag (beispielsweise 250, 200, 150, 100 oder 50 mg oder weniger pro Tag) eines dualen SGLT1/2-Inhibitors der Formel: 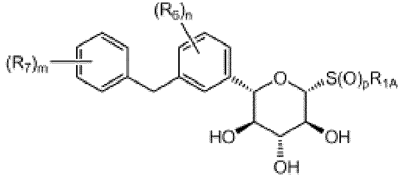 oder eines pharmazeutisch akzeptablen Salzes desselben, zur Verwendung bei der Verbesserung der kardiovaskulären oder metabolischen Gesundheit eines diabetischen oder prädiabetischen Patienten, der gegenwärtig einen DPP-4- Inhibitor einnimmt, wobei:
jedes R_{1A} unabhängig ein Wasserstoff, ein Alkyl, ein Aryl oder ein Heterocyclus ist;
jedes R₆ unabhängig ein Wasserstoff, ein Hydroxyl, ein Amino, ein Alkyl, ein Aryl, ein Cyano, ein Halogen, ein Heteroalkyl, ein Heterocyclus, ein Nitro, C≡CR_{6A}, OR_{6A}, SR_{6A}, SOR_{6A}, SO₂R_{6A}, C(O)R_{6A}, CO₂R_{6A}, CO₂H, CON (R_{6A}) (R_{6A}), CONH (R_{6A}), CONH₂, NHC(O)R_{6A} oder NHSO₂R_{6A} ist;
jedes R_{6A} unabhängig ein Alkyl, ein Aryl oder ein Heterocyclus ist;
jedes R₇ unabhängig ein Wasserstoff, ein Hydroxyl, ein Amino, ein Alkyl, ein Aryl, ein Cyano, ein Halogen, ein Heteroalkyl, ein Heterocyclus, ein Nitro, C≡CR_{7A}, OR_{7A}, SR_{7A}, SOR_{7A}, SO₂R_{7A}, C(O)R_{7A}, CO₂R_{7A}, CO₂H, CON(R_{7A}) (R_{7A}), CONH(R_{7A}), CONH₂, NHC(O)R_{7A} oder NHSO₂R_{7A} ist;
jedes R_{7A} unabhängig ein Alkyl, ein Aryl oder ein Heterocyclus ist;
m 1-4 ist;
n 1-3 ist;
p 0-2 ist; und
wobei jedes Alkyl, Aryl, Heteroalkyl oder jeder Heterocyclus gegebenenfalls mit einem oder mehreren aus einem Alkoxy, einem Amino, einem Cyano, einem Halogen, einem Hydroxyl oder einem Nitro substituiert ist; und wobei der DPP-4- Inhibitor Sitagliptin ist.

2. Eine Dosis zur Verwendung nach Anspruch 1, wobei die Verbesserung eine Absenkung des Spiegels der Nüchtern-Plasmaglukose (Nüchternblutzucker) des Patienten um mehr als etwa 50, 55 oder 60 mg/dl ist.

3. Eine Dosis zur Verwendung nach Anspruch 1, wobei die Verbesserung eine Absenkung des HbA1c- Spiegels des Patienten um mehr als etwa 1,0, 1,1 oder 1,2 Prozent ist.

4. Eine Dosis zur Verwendung nach Anspruch 1, wobei die Verbesserung eine Absenkung des Spiegels von Fructosamin im Plasma des Patienten um mehr als etwa 30, 40 oder 50 µmol/l ist.

5. Eine Dosis zur Verwendung nach Anspruch 1, wobei die Verbesserung eine Absenkung des Blutdrucks des Patienten ist.

6. Eine Dosis zur Verwendung nach Anspruch 1, wobei die Verbesserung eine Absenkung der Triglycerid- Spiegel des Patienten ist.

7. Eine Dosis zur Verwendung nach Anspruch 1, wobei der duale SGLT1/2- Inhibitor die Formel: 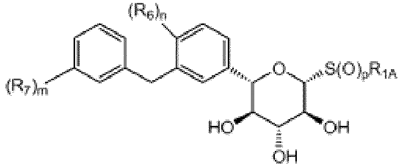 aufweist.

8. Eine Dosis zur Verwendung nach Anspruch 1, wobei der duale SGLT1/2- Inhibitor die Formel: 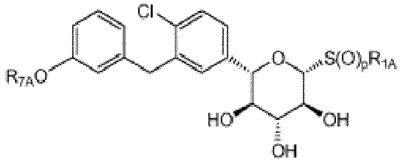 aufweist.

9. Eine Dosis zur Verwendung nach Anspruch 1, wobei der duale SGLT1/2- Inhibitor die Formel: 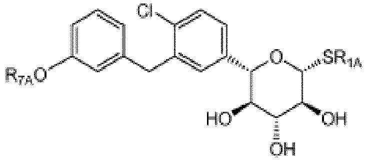 aufweist.

10. Eine Dosis zur Verwendung nach Anspruch 9, wobei R_{7A} ein Methyl oder ein Ethyl ist.

11. Eine Dosis zur Verwendung nach Anspruch 9, wobei R_{1A} ein Methyl ist.

12. Eine Dosis zur Verwendung nach Anspruch 11, wobei der duale SGLT1/2- Inhibitor (2S,3R,4R,5S,6R)-2-(4-Chlor-3-(4-ethoxybenzyl)phenyl)-6-(methylthio)tetrahydro-2H-pyran-3,4,5-triol ist.

## Revendications

1. Une dose de 300 mg par jour ou moins (par exemple, 250, 200, 150, 100 ou 50 mg par jour ou moins) d'un double inhibiteur SGLT1/2 de formule : 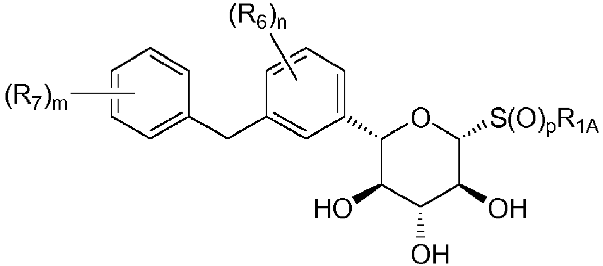 ou un sel pharmaceutiquement acceptable en dérivant, pour une utilisation dans l'amélioration de la santé cardiovasculaire ou de la santé métabolique d'un patient diabétique ou prédiabétique, prenant couramment un inhibiteur DPP-4, dans laquelle:
- chaque R_{1A} représente, indépendamment l'un de l'autre, un atome d'hydrogène, un groupe alkyle, aryle ou hétérocyclique;
- chaque R₆ représente, indépendamment l'un de l'autre, un atome d'hydrogène, un hydroxyle, un amino, un alkyle, un aryle, un cyano, un halogène, un hétéroalkyle, un hétérocycle, un groupe nitro, C≡CR_{6A}, OR_{6A}, SR_{6A}, SOR_{6A} SO₂R_{6A}, C(O)R_{6A}, CO₂R_{6A}, CO₂H, CON(R_{6A})(R_{6A}), CONH(R_{6A}), CONH₂, NHC(O)R_{6A}, ou NHSO₂R_{6A};
- chaque R_{6A} représente, indépendamment l'un de l'autre, un alkyle, un aryle ou un hétérocycle;
- chaque R₇ représente, indépendamment l'un de l'autre, un atome d'hydrogène, un hydroxyle, un amino, un alkyle, un aryle, un cyano, un halogène, un hétéroalkyle, un hétérocycle, un groupe nitro, C≡CR_{7A}, OR_{7A}, SR_{7A}, SOR_{7A} SO₂R_{7A}, C(O)R_{7A}, CO₂R_{7A}, CO₂H, CON(R_{7A})(R_{7A}), CONH(R_{7A}), CONH₂, NHC(O)R_{7A}, ou NHSO₂R_{7A};
- chaque R_{7A} représente, indépendamment l'un de l'autre, un alkyle, un aryle ou un hétérocycle;
m est 1-4;
n est 1-3;
p est 0-2; et
dans laquelle chaque groupe alkyle, aryle, hétéroalkyle ou hétérocyclique est éventuellement substitué par un ou plusieurs groupes alcoxy, amino, cyano, halogéno, hydroxyle ou nitro,
et dans laquelle l'inhibiteur DPP-4 est la sitagliptine.

2. Une dose pour une utilisation selon la revendication 1, dans laquelle l'amélioration est un abaissement du taux de glucose plasmatique à jeun du patient, de plus d'environ 50, 55 ou 60 mg/dl.

3. Une dose pour une utilisation selon la revendication 1, dans laquelle l'amélioration est un abaissement du taux de HbAlc du patient de plus d'environ 1,0, 1,1 ou 1,2 pourcent

4. Une dose pour une utilisation selon la revendication 1, dans laquelle l'amélioration est un abaissement du taux de fructosamine dans le plasma du patient, de plus d'environ 30, 40 ou 50 µmol/l.

5. Une dose pour une utilisation selon la revendication 1, dans laquelle l'amélioration est un abaissement de la pression sanguine du patient.

6. Une dose pour une utilisation selon la revendication 1, dans laquelle l'amélioration est un abaissement des taux de triglycérides du patient.

7. Une dose pour une utilisation selon la revendication 1, dans laquelle l'inhibiteur double SGLT1/2 présente la formule:

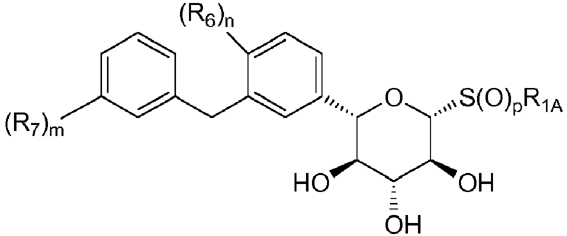

8. Une dose pour une utilisation selon la revendication 7, dans laquelle l'inhibiteur double SGLT1/2 présente la formule:

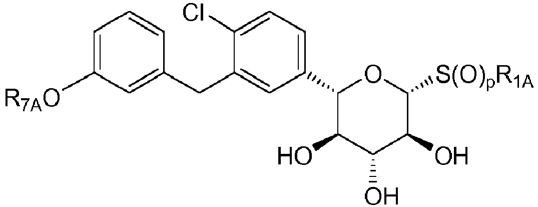

9. Une dose pour une utilisation selon la revendication 8, dans laquelle l'inhibiteur double SGLT1/2 présente la formule :

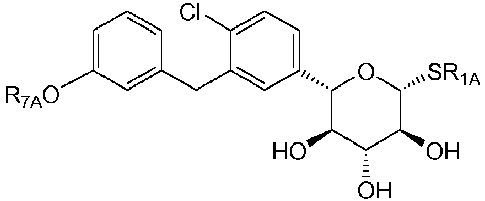

10. Une dose pour une utilisation selon la revendication 9, dans laquelle R₇ₐ est un groupe méthyle ou éthyle.

11. Une dose pour une utilisation selon la revendication 9, dans laquelle R_{1A} est un groupe méthyle.

12. Une dose pour une utilisation selon la revendication 11, dans laquelle l'inhibiteur double SGLT1/2 est le (2S, 3R, 4R, 5S, 6R)-2-(4-chloro-3-(4-éthoxybenzyl)phényl)-6-(méthylthio)tétrahydro-2H-pyran-3,4,5-triol.
